# EUROPEAN PATENT SPECIFICATION

(11) **EP 1 112 091 B1**
(45) Date of publication and mention of the grant of the patent: **17.09.2003**
(21) Application number: 99930271.4
(22) Date of filing: 15.06.1999
(51) Int. Cl.: A61L 2/00, A61L 2/02

(54) **RAPID CRYOBARIC STERILIZATION AND VACCINE PREPARATION**
SCHNELLE TIEFTEMPERATUR-DRUCKSTERILISATION UND IMPFSTOFFHERSTELLUNG
STERILISATION CRYOGENIQUE RAPIDE ET PREPARATION DE VACCIN

(43) Date of publication of application: 04.07.2001
(73) Proprietor: BBI Bioseq, Inc., Woburn, MA 01807-6307 (US)
(72) Inventor: LAUGHARN, James, A., Winchester, MA 01890 (US); BRADLEY, David, W., Bedford, MA 01730 (US); HESS, Robert, A., Cambridge, MA 02318 (US)
(74) Representative: Grund, Martin, Dr.
(86) International application number: PCT/US99/13461
(87) International publication number: WO 00/048641

(56) References cited:
- EP-A- 0 410 207
- WO-A-99/22868
- US-A- 3 445 568
- US-A- 4 164 538
- US-A- 5 316 745
- US-A- 5 871 900
- HASHIZUME C ET AL: "KINETIC ANALYSIS OF YEAST INACTIVATION BY HIGH PRESSURE TREATMENT AT LOW TEMPERATURES" BIOSCIENCE BIOTECHNOLOGY BIOCHEMISTRY,JP,JAPAN SOC. FOR BIOSCIENCE, BIOTECHNOLOGY AND AGROCHEM. TOKYO, vol. 59, no. 8, 1 August 1995 (1995-08-01), pages 1455-1458, XP000530302 ISSN: 0916-8451
- HAYAKAWA ET AL.: 'Oscillatory Compared with Continuous High Pressure Sterilization on Bacillus stearothermophilus Spores' JOURNAL OF FOOD SCIENCE vol. 56, no. 1, 1994, pages 164 - 167, XP000440407
- HASHIZUME ET AL.: 'Kinetic Analysis of Yeast Inactivation by High Pressure Treatment at Low Temperatures' BIOSCI. BIOTECH. BIOCHEM. vol. 59, no. 8, 1995, pages 1455 - 1458, XP000530302

## Description

### Cross Reference To Related Application

This application claims priority from U.S. Serial Number 09/097,852, filed June 15, 1998, and from U.S. Serial Number 09/165,829, filed October 2, 1998, both of which are incorporated herein by reference in their entirety.

### Background of the Invention

The invention relates to methods for sterilizing materials and preparing vaccines.

Various methods and devices exist for the sterilization, decontamination, or disinfection of biological and non-biological materials. These methods include thermal destruction (e.g., burning), heat sterilization, irradiation (e.g., ultraviolet or ionizing irradiation), gas sterilization (e.g., using ethylene oxide), photosensitization, membrane sterilization, and the use of chemical disinfectants (e.g., formaldehyde, glutaraldehyde, alcohols, mercury compounds, quaternary ammonium compounds, halogenated compounds, solvent/detergent systems, or peroxides).

Heat sterilization (e.g., autoclaving) is often used, for example, for sterilizing medical solutions prior to use in a patient. Heat sterilization typically requires heating a solution to 121°C for a minimum of 15 minutes under pressure in an autoclave, maintaining the heat and pressure conditions for a period of time sufficient to kill bacteria, fungi, and protists and inactivate viruses in the solution.

Many reusable medical articles and materials are not suitable for disinfection or sterilization in an autoclave. For example, plastic parts on medical devices, hemodialyzers, and fiber optic devices are commonly sterilized by chemical germicide treatment. In general, germicides require up to several hours of treatment for the inactivation of microorganisms.

To ensure sterility in pharmaceutical production, gas sterilization is often employed. However, gas sterilization (e.g., using ethylene oxide) can be time-consuming, requiring prehumidification, heating, and evacuation of a sample chamber, followed by treatment with high concentrations of the gas for up to 20 hours at a time.

When properly used, traditional disinfectants can inactivate vegetative bacteria, certain fungi, and lipophilic or medium-sized viruses. However, these disinfectants often do not arrest tubercle bacillus, spore-forming bacteria, or non-lipophilic or small-sized viruses.

Another method for lysing cells, and thereby sterilizing a sample is described in *Microbiology* (Davis et al., Harper & Row, Hagerstown, MD, 1980). This procedure of freezing and thawing the sample is believed to exert its effect through formation of tiny pockets of ice within the cells when a suspension of bacteria is frozen. The ice crystals and the high localized concentrations of salts both cause damage to the bacteria. A single freezing event is generally sufficient to kill only some of the bacteria, but repeated freeze-thaw cycles result in a progressive decrease in viability. Lethality is correlated with slow freezing and rapid thawing.

Traditional freeze-thaw methods are limited in the speed of the freeze-thaw cycle by the time needed to transfer heat to and from the center of the sample to effect phase changes. The equilibrium rate is particularly slow in the case of large volume samples (e.g., about 100 ml or larger). Sterilization efficiency of the traditional methods is limited by the impracticality of performing a large number of freeze-thaw cycles by those methods.

Traditional methods of food preservation include pasteurization, in which a food is held at an elevated temperature for a period of time. US-Patent No. 3,445,568 discloses an electrohydraulic process, wherein microorganisms suspended in a liquid medium are subjected to one or more steep pressure or shock waves in order to destroy the viability of the microorganisms without destroying their antigenicity. Furthermore, it is disclosed that some combination of chemicals, UV radiation, heat, pressure shock, and turbulence can destroy microorganisms. US-Patent No. 5,316,745 describes an ultra high pressure sterilizing apparatus and a method to sterilize a material, wherein said material is exposed to high pressure levels and reduced pressure levels. Hashizume C. et al. (Biosci. Biotech. and Biochem., 59 (8), 1455-1458, 1995) have shown that high pressure treatment applied to food sterilization at lower temperatures has a greater effect with smaller pressure without destroying the original taste and flavor. Furthermore, that sterilization takes place when high pressure destroys the natural structures of biological macromolecules, i.e. proteins and lipids of microorganisms.

There is an ongoing need to develop improved methods for sterilization, particularly for the inactivation of viruses and other microbes in materials that also contain proteins having activity that one desires to retain, such as clotting factors, antibodies, blood enzymes (e.g., lipases, phosphatases), and growth factors. The development of methods for inactivation of non-encapsulated viruses is especially challenging, since the outer coats of such viruses generally include proteins similar to the proteins one wishes to retain.

### Summary of the Invention

The invention is based on the discovery that biological and non-biological materials can be sterilized, decontaminated, or disinfected by repeatedly cycling between relatively high and low pressures. Pressure cycling can be carried out at low, ambient, or elevated temperatures (e.g., from about -40°C to about 95°C). New methods based on this discovery can have applications in, for example, the preparation of vaccines, the sterilization of blood plasma *or* serum, the decontamination of military devices, food and beverage production, and the disinfection of medical equipment. The new methods can also be incorporated into production processes or research procedures.

In general, the invention features a method for sterilizing a material. The method includes the steps of providing a material at an initial pressure (e.g., 1 atm) and temperature (e.g., 25°C, lower temperatures such as 0°C, - 5°C, -25°C, -40°C or lower) ; coding the material to a temperature Te, wherein Te is -40 to 10°C increasing the pressure to an elevated hydrostatic pressure sufficient to inactivate at least some (e.g., at least about 10%, 25%, 50%, 75%, 90%, 95%, 99%, or even substantially all) microbes contained in the material (e.g., in the range of about 5,000 psi [34.5 MPa] to about 95,000 psi [655.0 MPa], or in the range of about 10,000 psi [68.9 MPa] to about 75,000 psi [517.1 MPa] or in the range of about 95,000 psi [655 MPa] to about 150,000 [1034.5 MPa]; and subsequently decreasing the pressure to a reduced pressure, which may be about the same as, less than, or greater than the initial pressure (e.g., around 1 atm) and repeatedly cycling the pressure between the elevated pressure and the reduced pressure, to provide a sterilized material (i.e., a material having a reduced titer of microbes).

In some cases, the material includes a protein. In such cases, it may be desirable that the elevated pressure be insufficient to irreversibly denature proteins during the time that the pressure is at an elevated level. One skilled in the art would understand that "denaturing a protein" means denaturing a sufficient amount of the protein so as to reduce or destroy the usefulness of the protein for a particular application. Typically, irreversibly denaturing more than about 50% of the protein molecules in the sample would render the sample an unsatisfactory source of the protein. In some cases, however, retention of even 10% or less of the protein activity is adequate.

The material can be chilled to a subzero temperature (e.g., from about -40°C to about 0°C, especially between about -20° and about -5) either before or after the pressure is increased. The temperature can be subsequently increased, either before or after the pressure is decreased.

The pressure is repeatedly cycled (e.g., 2, 3, 5, 10, or even 100 or more times) between the elevated pressure and the initial pressure. Such cycling can be carried out at the initial temperature, at a low temperature (e.g., subzero temperatures such as between -40°C and 0°C, or between -20°C and -5°C), or while the material is being cooled to a low temperature. The timing of the cycles may be such that the temperature of the material is allowed to equilibrate (e.g., to the temperature of the walls of a reaction vessel in which the method is carried out) prior to each cycle.

In some cases, a material at low temperature can be in the solid (i.e., frozen) state at the initial pressure, but in the liquid (i.e., molten, or thawed) state at the elevated pressure. In such cases, pressure cycling causes concomitant freeze-thaw cycling. The temporal pattern of pulsation can, optionally, be altered. During each cycle, the pressure is alternately raised and then lowered. The ratio of the time at high pressure to the time at low pressure is termed as the "pulsation pattern ratio." A pulsation pattern ratio greater than 1:1 (e.g., 2:1 or more) can give optimal inactivation of contaminants in most cases, whereas a pulsation ratio less than 1:1 can give greater retention of properly folded, sensitive proteins.

The material being sterilized can be, for example, a biological sample, blood plasma, therapeutic and/or diagnostic products derived from blood plasma, biological fluids, medical fluids, medicaments, research solutions and reagents, serum, living tissue, medical or military equipment, a foodstuff, a pharmaceutical preparation, or a vaccine. The material being sterilized can be initially infected with, contaminated with, or otherwise contain, for example, one or more of a bacterium, a virus, a fungus, a protist, a spore former, a protozoan parasites, malaria-inducing organisms, giardia, or virally infected cells.

The invention also features a method for inactivating a virus in a material. The method includes the steps of providing a material at an initial pressure and temperature; and exposing the material to repeated pressure cycles (e.g., 2 to 100 cycles, more than about 3, 10, 50, 100, 1,000, or 10,000 cycles). Each pressure cycle includes the steps of increasing the pressure to an elevated pressure (e.g., between about 10,000 psi [68.9 MPa] and about 120,000 psi, [827.4 MPa] between about 40,000 psi [275.8 MPa] and about 100,000 psi [689.5 MPa], or between about 70,000 psi [482.6 MPa] and about 90,000 psi) [620.5 MPa], maintaining an elevated pressure for a time period tₑ, decreasing the pressure to a reduced pressure, and maintaining the material at a reduced pressure (e.g., a pressure less than the elevated pressure, and less than, equal to, or greater than the initial pressure), for a time period tᵢ. The elevated pressure is sufficient such that each cycle inactivates at least some (e.g., at least about 1%, 5%, 10%, 25%, 50% or more) of the virus in the material when the elevated pressure is maintained for time tₑ (e.g., between about 0.5 and about 300 seconds, or between about 10 and about 30 seconds).

In some cases, the material includes a protein. In such cases, the elevated pressure can be a pressure that would be sufficient to irreversibly denature substantially all (e.g., 50%, 75%, 90%, 95% or more) of the protein if the elevated pressure were maintained for a time substantially longer than tₑ (e.g., 2, 3, 5, 10, or 100 times tₑ, or more), but is insufficient to irreversibly denature the protein when the elevated pressure is maintained for only time period tₑ or less.

The protein can be, for example, a blood clotting factor (e.g., factor VII, VIII, IX, XI, and or XIII), an immunoglobulin (e.g., IgG, IgM), a monomeric protein, a multimeric protein, or a mixture of proteins.

The virus can be an encapsulated or non-encapsulated virus (e.g., human parvovirus B19, porcine parvovirus, bovine parvovirus, human immunodeficiency virus, herpes simplex virus, hepatitis A virus, bacteriophage MS2, or transfusion transmitted virus).

The method can also include the step of cooling the material to a temperature Tₑ, (e.g., about -40°C to about 10°C, or about -25°C to about -10°C), prior to exposing the material to the elevated pressure.

Any of the new methods described above can also be used to produce vaccines against specific microbes. For example, a suspension of microbial cells can be obtained, sterilized by one of the new methods (e.g., the method that involves pressure cycling, and potentially freeze/thaw cycling, at a subzero temperature), and combined with an adjuvant to virus, herpes simplex virus, hepatitis A virus, bacteriophage MS2, or transfusion transmitted virus).

The method can also include the step of cooling the material to a temperature Tₑ, (e.g., about -40°C to about 10°C, or about -25°C to about -10°C), prior to exposing the material to the elevated pressure.

Any of the new methods described above can also be used to produce vaccines against specific microbes. For example, a suspension of microbial cells can be obtained, sterilized by one of the new methods (e.g., the method that involves pressure cycling, and potentially freeze/thaw cycling, at a subzero temperature), and combined with an adjuvant to produce a vaccine. If there are toxins present in the suspension, these can removed (e.g., after the sterilization step).

In some cases, it can be useful to include an additive such as a phase-change catalyst (e.g., glass particles), a protein-stabilizing agent (e.g., a sugar, glycerol, a hydrophilic polymer, a cyclodextrin, a caprylate, acetyl tryptophanoate, polyethylene glycol, anti-oxidant, or a protein specific ligand), or a nucleic-acid binding compound (e.g., a photosensitizer such as a psoralen) in conjunction with the material to be sterilized. Some such additives can subsequently be removed by centrifugation or filtration, if necessary.

In another embodiment, the invention features an apparatus for sterilizing a material. The apparatus includes a pressurization vessel adapted to transmit an external pressure to a material within itself. The vessel needs to be capable of withstanding an elevated pressure (e.g., pressures encountered in the practice of any of the new methods described above), must be capable of fitting in a pressure cycling apparatus (e.g., such as psi); due to the short duration of the pressure cycles (e.g., between about 1 s and about 300 s), and in some cases the low temperatures (e.g., between about -10°C and about -40°C), however, the new methods avoid causing excessive irreversible denaturation of the proteins in the materials sterilized thereby.

In yet another embodiment, the invention features another method for sterilizing a material that includes proteins. The method includes the steps of providing the material at an initial pressure; adding one or more protein-stabilizing reagents (e.g., sugars such as glucose; glycerol; a hydrophilic polymer; a cyclodextrin; a caprylate; acetyl tryptophanoate; polyethylene glycol; an anti-oxidant; or a protein specific ligand) to the material; increasing the pressure to an enhanced pressure (e.g., about 10,000 [68.9 MPa] to 70,000-80,000 psi [482.6 - 552 MPa], depending on the stability of the protein); incubating the material for a time sufficient for sterilization to occur without substantial loss of protein function; and restoring the pressure to the initial pressure, to provide a sterilized material. Again, the pressure may be repeatedly cycled.

In any of the above methods, the material to be sterilized can be provided in its final packaging, the packaging being able to transmit pressure without rupture. For example, the packaging can be hermetically sealed in flexible plastic (e.g., PVC or polyethylene). Alternatively, the packaging can be a syringe and the pressure can be transmitted via a plunger.

The invention also features a method for pressurizing an infectious material. The method includes the steps of charging the material into a container adapted to transmit an external pressure to the material; submerging the container in a sterilizing chemical solution (e.g., containing an oxidizing agent, an alcohol, urea, a guanidinium salt, an acid, or a base); and pressurizing the material within the container.

In any of the above methods, the pH of the material to be sterilized can optionally be adjusted to greater than about 10 (e.g., between 10 and 14, or between 11 and 12) or less than about 4 (e.g., between 0 and 4, or between 2 and 3), prior to increasing pressure. Such pH adjustment can be useful, for example, for inactivation of pH-sensitive microbes (e.g., parvovirus) or microbes in materials that include proteins that are resistant to extremes of pH (e.g., IgG), can accelerate the sterilization methods, and can, in some cases, allow the methods to be carried out at lower pressures overall.

As used herein, the term "subzero temperature" means a temperature lower than 0°C (e.g., -1°C, -5°C, -10°C, -20°C, or lower). All temperatures herein are in degrees Celsius unless otherwise stated, and are simply denoted by "°C". Units of pressure herein are expressed in pounds per square inch (psi) or in atmospheres (atm). 1 atmosphere is about 14.5 psi, 1 bar, or 101.3 kilopascals.

A "cryobaric process" is a process that involves at least one pressure change carried out at a subzero temperature. In some cryobaric processes, the pressure is cycled between two pressures (e.g., about 14.5 psi [0.1 MPa] to about 5,000 psi [34.5 MPa], 35,000 psi [241.5 MPa], 70,000 psi [483 MPa], 80,000 psi [552 MPa], 100,000 psi [689.5 MPa], or higher) while the temperature is either maintained at a subzero temperature or varied within a subzero temperature range.

The terms "sterilize", "disinfect", "inactivate", and "decontaminate" are used interchangeably herein, unless otherwise demanded by the context. It should be noted that "sterilization" (killing of all organisms) may not be synonymous in certain operations with "decontamination" when the contaminant is non-living, such as a protein or prion.

The new methods provide several advantages. For example, the methods can be carried out at subzero temperatures (e.g., between about -40°C and 0°C). Pressure cycling carried out at subzero temperatures can advantageously induce oscillation between different phases of water within or outside the cells or vesicles of biological contaminants. The transition between the liquid and solid states can create physical stress on membranes, walls, and vesicles, thereby facilitating the intended processes. The range of subzero temperatures generally used in the new methods is easily accessed with relatively inexpensive equipment (e.g., commercial chilling devices) that is readily available in a range of shapes and sizes to fit a specific need. Similarly, the range of pressures required for the standard operation of the methods (e.g. from about 14.5 psi [0,1 MPa] to about 30,000 psi [206.8 MPa], 70,000 psi [482.6 MPa] 80,000 psi [552 MPa], 100,000 psi [690 MPa], or higher) can be generated by devices as described in PCT US97/03232.

An apparatus for sterilization of a material by a cryobaric process will generally include a chamber for containing the material, the chamber being capable of operation at a selected elevated pressure, and a system for controlling, altering, or regulating the temperature and pressure within the chamber. The apparatus can allow for increasing and decreasing the pressure at a rapid rate sufficient to avoid denaturation of proteins during the pressurization and depressurization steps of the pressure cycles. Optimal rapidity is dependent, for example, on the temperature used in the process; generally, higher rates are required at higher temperature. The apparatus will also provide systems for removing a sterilized material in an aseptic manner from the chamber. Additionally, a typical sterilization apparatus for use with the new methods can include a variety of controls, regulators, and temperature, and/or pressure sensors. The pressurizing medium can be, for example, a water/ethylene glycol solution or other non-freezing solution or a solid such as powdered talc.

The devices necessary for carrying out the new methods can be easily adapted to conform the requirements of particular applications. For example, a small, portable device can be obtained, thereby allowing sterilization or decontamination procedures to be carried out in the field (e.g., by paramedics or military medical personnel).

Variation of temperature can also aid lysis of microbes. Temperature, pressure, cycle count, or cycle frequency can be varied to maximize the effectiveness of the sterilization processes.

The new methods are very rapid. For example, the pressure can be cycled at a frequency of about 1 mHz to about 10 Hz, typically allowing the entire sterilization process to be completed within minutes.

The new methods allow pathogenic organisms in a material to be neutralized without concomitant denaturation of proteins. The new methods can avoid denaturation, which often occurs upon sterilization of biological materials.

Rapid and economical sterilization is achievable with a minimum of protein destruction or denaturation. The new methods can thus be advantageously used for the production of highly active vaccines. These vaccines can be superior to vaccines produced at higher temperatures, since high temperatures can cause disruption of both covalent and noncovalent bonds in proteins, and can lead to a greater degree of irreversible denaturation than the methods claimed here. High temperatures may also lead to covalent linkage of proteins to small molecules such as oxidized glucose. Another advantage of the invention is that it can be used to inactivate viruses and bacteria in patient samples prior to analysis. Laboratory workers can be subject to risk of acquiring infection (e.g., from viruses such as human immunodeficiency virus and hepatitis B virus) when handling samples of tissues, cells or fluids taken from patients for analysis. To avoid this risk, such samples can be collected into a container that has been designed to insert into a pressure cycling apparatus. A suitable container might be compressible, or might have a piston or plunger to transmit pressure. The methods of the invention can then be carried out on the sample in the container, without exposure of the sample prior to pathogen inactivation. In one example, blood collected in a syringe is capped and the capped syringe is placed inside a pressure chamber filled with a sterilizing pressure-transmitting medium such as 70% ethanol. The sample is then treated with a method of the invention and removed for analysis.

Other advantages of the new methods include the avoidance or reduction of the need for addition of chemical additives to blood fractions; scalability of the process from single units to large, pooled samples or to continuous, on-line processes; and elimination of side effects of thermal inactivation processes on protein components.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

Other features and advantages of the invention will be apparent from the following detailed description, and from the claims.

### Detailed Description

The invention provides a method by which a material can be sterilized or decontaminated by high pressure in the range of about 5.000 [34.5 MPa] to 95,000 psi [655 MPa] preferably in the range of 30,000 [206.8 MPa] to 75,000 psi [517.1 MPa]. The material is adjusted, either before or after pressurization, to a particular temperature which is both compatible with preserving the desirable properties of the material, and which also allows destruction of the contaminants.

Although the temperature, pressure, number and duration of cycles, and relative timing of pressure and temperature changes can vary, the new methods are in general carried out according to the following procedure: A material is provided at initial pressure (e.g., atmospheric pressure, 14.5 psi) and temperature (e.g. ambient temperature, 25°C, or a refrigerated temperature such as 0°C to 4°C, or a frozen temperature such as -80°C to -20°C). The material is then pressurized to an elevated pressure. The pressure can then be cycled repeatedly between the elevated pressure and ambient pressure. The material can be produced in a frozen state after the final depressurization, or can be warmed to 0°C or higher before depressurization to produce a non-frozen, sterilized product.

The above processes can also be used in conjunction with methods in which a known static pressure and/or a particular pressure is maintained for a given time. In some cases, the pressure may be maintained at a greatly elevated level for an amount of time sufficient for microbe inactivation but insufficient for irreversible protein denaturation.

If a phase change is involved in any sterilization process, a catalyst may be added to accelerate the change. For example, the presence of finely-divided glass or other materials can provide nucleation sites for freezing of a sample or material.

### General

Applications of the new methods include the sterilization of materials such as blood plasma from donors (e.g., for use in transfusions), the sterilization of purified or partially purified therapeutic and diagnostic proteins derived from blood, medical samples (blood, urine, fecal, hair, biopsy, or other tissue samples), pharmaceuticals (e.g., biosynthetic antisense drugs), biopharmaceuticals, and transgenically produced proteins. The new methods can be used to arrest the proliferation of viruses in incubated materials or to sterilize such materials. The new methods can also be used to ensure sterility of cosmetics, pharmaceutics, and industrial products.

Examples of microbes that can be inactivated by the new methods include both hydrophilic and lipophilic viruses; nearly any bacteria, including, for example, *Staphylococci, Micrococci, Pyogenic streptococci,* diphtheroids (e.g., lipophilic, non-lipophilic, anaerobic diphtheroids such as *Propiobacterium*), gram-negative enteric bacilli (e.g., *Escherichia, Enterobacter, Klebsiella, Proteus, Serratia*), *Neisseria,* aerobic spore formers, mycobacteria; fungi, including, for example, yeast, *Pityrosporum ovale, Pityrosporum orbiculare, Candida albicans, Candida parapsilosis, Torulopsis glabarata,* and filamentous dermatophytic species; protists and lower multicellular organisms, including protozoan parasites; and helminth parasites; malaria-inducing organisms; giardia; and virally infected cells.

Examples of viruses that can be inactivated by the new methods include both DNA and RNA viruses, such as human parvovirus B19 ("B19"), porcine parvovirus ("PPV"), bacteriophage MS2 ("MS2"), and hepatitis A virus (HAV). Both B19 and HAV are small (about 15-30 nm), do not possess an outer envelope composed of lipids (non-enveloped), are resistant to heat and chemical treatment, and are difficult to remove by nanofiltration. PPV and MS2 are similarly resistant viruses that can serve as research models for the human pathogens B19 and HAV. Enveloped viruses (e.g., human immunodeficiency virus, hepatitis B virus, and hepatitis C virus) are also potential targets of the new methods. Currently uncharacterized viruses, such as some newly-recognized forms of hepatitis virus and transfusion transmitted virus can also be vulnerable to the new methods.

Plasma pools often contain hepatitis C virus (HCV). Procedures for producing blood products can thus benefit from a process that inactivates HCV and other viruses. Human parvovirus B19 (B19), is another common contaminant of plasma. Hepatitis A virus (HAV) contaminants are less common, but still troublesome. Both B19 and HAV are small (about 15-30 nm), do not possess an outer envelope composed of lipids (non-enveloped), are resistant to heat and chemical treatment, and are difficult to remove by nanofiltration. Enveloped viruses (e.g., HIV, HBV, HCV) are also potential targets of the new methods. Currently uncharacterized viruses, such as some newly-recognized forms of hepatitis virus and transfusion transmitted virus (TTV) can also be vulnerable to the new methods. In addition, prion-based infectious agents such as transmissible spongiform encephalopathies are difficult to screen and to inactivate. However, because the methods of the invention can, under suitable conditions, induce protein unfolding, it may be possible to inactivate such agents by the methods of the invention.

Due to the possibility that disrupted virus particles can re-assemble after pressure treatment, it can be desirable to irreversibly degrade the nucleic acids contained in the virus. Moderately high pressures (e.g. 20,000 psi [138 MPa] to 60,000 psi [414 MPa]) can disrupt complexes of nucleases and their endogenous inhibitors. The activated nucleases can serve to degrade nucleic acids and thereby enhance irreversible inactivation of viruses. Additionally, moderate pressure can accelerate the activity of uninhibited enzymes. The process may be enhanced by the addition of nucleases. It is desirable in some cases to add a magnesium independent nuclease, as in the treatment of citrated plasma, since the citrate can bind magnesium ions thus inhibiting magnesium-dependent nucleases.

Alternatively, much higher pressures (e.g. 50,000 psi to 150,000 psi [345 - 1035 MPa]) can be used for sterilization of materials that are pressure-stable, such as small molecule pharmaceuticals or thermostable proteins. A pressure-cycling freeze-thaw sterilization method (e.g., a method that takes advantage of the cyclic formation of high pressure ice such as ice III, ice IV, ice V or ice VI) may also be used.

When the biological contaminants are relatively pressure stable and the material contains labile proteins that need to be retained, a variation of this method can be used. In this variation, the pressure is increased rapidly (e.g., in less than 5 seconds, or less than 1 second) to a very high maximum pressure (e.g., 100,000 psi [690 MPa] or higher), and held at high pressure only briefly (e.g., less than 5 seconds). The pressure and time are chosen to provide a high degree of microbe inactivation, but the time is brief enough that proteins denatured by the elevated pressure conditions do not have time to aggregate into irreversible complexes to a sufficient extent before they refold into their native forms. The pressure is then rapidly released (e.g., in less than 5 seconds, or less than 1 second). The inactivation of microbes such as viruses can proceed at a much greater rate than the irreversible aggregation or denaturation of protein.molecules.

Carrying out the pressure cycling at low temperature can further improve retention of active protein, by slowing the rate of aggregation and increasing the rate of protein refolding. Under certain conditions of high pressure and low temperature (e.g., 100,000 psi [690 MPa] and -20°C), high pressure ice (i.e. ice V or ice VI) can form. Proteins that are trapped in the lattice structure of the high pressure ice are less likely to aggregate. The high pressure ice takes a finite amount of time to melt, this time being sufficient for the proteins in the material to refold while trapped in the solid phase. The addition of glucose can also increase the rate of protein folding.

Pressure has also been shown to increase the activity of numerous enzymes. For example, RNase activity is accelerated by elevated hydrostatic pressure. This effect can be exploited in conjunction with the new methods for the inactivation of viruses. RNA viruses are readily degraded following high pressure treatment.

### Blood transfusion

The new methods can be used to improve the safety of blood transfusions. Plasma protein products such as intravenous immunoglobulin ("IVIg"), factors VII, VIII, IX, XI, and XIII, albumin, von Willebrand's factor, fibrinogen, antithrombin III, protein C, Cl-inhibitor, alpha-1 antitrypsin, and fibrin sealant are needed, for example, by hemophiliacs, cancer patients, and kidney dialysis patients. However, viruses present in blood products can present a risk for patients in need of those products. Even using new filtration techniques that eliminate many cells, certain bacteria and viruses can remain in the products.

Ordinarily, blood plasma is isolated by obtaining a blood sample (e.g., collected in a tube containing an anticoagulant), centrifuging the sample in a plasma separation tube, and decanting the plasma from the precipitate in the tube. Although this method frees the plasma from the bulk of the cells, some cells inevitably remain in the plasma. If the remaining cells include, for example, bacteria or viruses, diseases can be spread by transfusion. The new methods can be carried out on the plasma obtained from the above decanting method. The contaminants that remain in the plasma can be inactivated by the new methods.

### Sterilization of Reagents and Media

The new methods can also be used to sterilize industrial products. For example, bovine serum is often used in molecular biology laboratories for cell cultures. Microbial contamination of the source stock material from the supplier occurs infrequently; when it does happen, however, the economic costs and time delays can often be significant. Current methods for sterilization of fetal calf serum (e.g., heat or filtration) can inactivate functionally important proteins (e.g., growth hormones) and also cause variability from lot to lot. Moreover, even if the source stock material is initially sterile, it can become inadvertently contaminated upon opening in the laboratory. The new methods can be used in either a production process (e.g., batch or continuous) or used in individual laboratories for pretreatment of serum or other media prior to initiation of an experiment.

### Vaccine Production

Vaccines are typically prepared by subjecting a solution of cultured viruses to an inactivating treatment (e.g., heating, or addition and removal of chemical denaturants).

A successful vaccine preparation method should ideally result in a high degree of viral inactivation, but should allow the product to retain its ability to stimulate a protective immune response in the patient. High pressure procedures are well suited to meet the criteria needed for successful vaccine production: since cold, pressure-denatured proteins retain a more native-like structure than do heated or chemically-denatured proteins, pressure inactivated viruses can thus be more immunogenic. Pressure-denatured proteins are also less likely to aggregate, thereby providing higher yields of vaccine. The pressure-inactivation methods described herein can be economical on a large scale since there are generally no chemicals to add or remove and, unlike heat, pressure can be transmitted rapidly through a large sample.

The specific conditions necessary for vaccine production can vary depending on the particular microbe to be inactivated. For example, in the case of spore forming organisms, an optional pretreatment with low pressure and moderate temperature (e.g. 10,000 psi [68.9 MPa] and 40°C) can be applied to cause the spores to germinate. The germinated spores can then be inactivated by the methods of the invention.

### Pressure enhanced photosensitization of nucleic acids:

A method of sterilization has been described, wherein the product to be sterilized is mixed with a chemical agent that can preferentially bind to DNA or RNA and react with the nucleic acid (Radosevich, "Seminars in Thrombosis and Hemostasis," Vol. 24, No. 2, pp. 157-161, 1998). In some cases, light is used to activate the chemical moiety. Disadvantages of such a method can include collateral damage to the desired molecular components of the product to be sterilized (e.g., via non-specific reaction with chemicals or irradiation, or by imperfect or slow penetration of the inactivating chemical to the interior of the microbe). The application of elevated pressures can substantially overcome these problems by permeabilizing cells and viruses to allow entry of the inactivating chemicals. Elevated pressures can also enhance the affinity and selectivity of the molecules for the nucleic acids, thereby allowing the use of lower chemical concentrations or lower amounts of irradiation. Thus, a faster, less expensive, and more efficient method is obtained.

An apparatus for the execution of the photochemical method can include a high pressure flow-through system such as described in PCT Appln. US96/03232, having a reaction chamber that includes at least one pressure-resistant window which can be made of a material such as quartz or sapphire, and a device for irradiation of the material through that window. The flow of liquid is such that all of the material passes through the irradiated area. The material can then be collected aseptically. The material can be introduced into the reaction chamber at high pressure or at low pressure, and then pressurized prior to irradiating.

### Chemical Inactivation of Viruses

A variety of chemicals (e.g. iodine, ethyleneimine, ascorbic acid, thiophosphamide, congo red, paraformaldehyde) can be used to sterilize solutions containing labile proteins. Use of such chemicals can have negative effects, however, including slow inactivation, potential for protein damage, or the inability of compounds to penetrate to the interior of the microbe. Elevated pressure can enhance the sterilization activity of these chemicals without exacerbating the negative effects (e.g., by increasing the chemicals' effectiveness against heat stable non-encapsulated viruses such as parvovirus and hepatitis A virus.

### Stabilization of proteins and enzymes

In some cases, it may be desirable to sterilize a solution or other material containing an unstable protein that would be irreversibly denatured at the pressure necessary for the sterilization procedures described above. In these cases, a stabilizing agent (e.g., amino acids such as amino acids, such as glycine, or specific ligands of proteins in the mixture, ligands of proteins to be recovered, or sugars such as glycerol, xylose, or glucose) can be added to the material prior to pressurization. For example, the human serum albumin stabilizers caprylate and acetyl tryptophanoate can be added to blood plasma samples, and the plasma samples can then be subjected to the cryobaric sterilization process without excessive destabilization of specific plasma proteins. The stabilizer can then be removed by standard methods (e.g. dialysis, filtration, chromatography).

### Pressure treatment of infectious materials

Hydrostatic or pulsating pressure can be a useful tool for sterilization, cell and virus disruption, and nuclease inactivation for materials that may potentially contain agents of infectious disease. Moreover, general safety considerations call for the prevention of infection of the persons handling the material and the avoidance of contamination of other materials. One way to prevent such contamination is to use a sterilizing solution (e.g., 10% CLOROX® bleach; 70% ethanol; concentrated urea; or a guanidinium salt) or an oxidizing agent as a pressurizing medium.

For example, the material can be placed inside an enclosed and flexible container, which can then be immersed in the chemical sterilizing solution. The solution can then be sealed inside of a second, chemically inert container (i.e., to keep it from contacting the metal parts on the inside of a pressurization chamber). An inert pressurizing medium can then be used to fill the volume between the inside of pressurization chamber and the container holding the material and sterilizing solution. The container that holds the sterilizing solution can be, for example, a plastic bag, a screw top plastic container, a capped syringe, or a shrink wrapping.

The invention will be further described in the following examples, which do not limit the scope of the invention described in the claims.

### Examples

### Example 1: Cryobaric Deactivation of Lambda Phage

For each of 4 samples, 2.5 µl Lambda phage stock (5 x 10¹¹ pfu/ml) was diluted 100-fold by addition of about 248 µl calf serum. A fifth 2.5 µl lambda phage stock sample was diluted 100 fold in suspension medium. Two phage-serum samples (250 µl) were frozen by immersing tubes in an ethanol-dry ice bath; three other samples were placed on ice.

One of the frozen phage-serum samples was cycled between 36,000 psi [248.4 MPa] (30 seconds) and 14.7 psi [0.101 MPa] (30 seconds) for 5 minutes. During pressure treatment, the temperature of this sample was maintained at -10°C. The remaining frozen sample was left at 14. 7 [0.101 MPa] psi throughout the experiment as a control. When this experiment was repeated, a dry-ice sample was warmed to -10°C for 5 minutes. A third phage-serum sample was pressurized to 36,000 psi [248.4 MPa] for 10 minutes at 23°C. The two remaining samples, used as positive controls, were a phage-serum sample incubated at 23°C for 10 minutes with no pressure treatment and a phage-suspension medium (i.e., 0.0496 M sodium chloride, 4.06 mM magnesium sulfate, 50 mM Tris-Cl pH 7.5, and 1.0 g/l gelatin) with neither pressure nor temperature treatment, respectively. After pressure treatment, 10², 10⁴, and 10⁶ fold dilutions of phage samples were made in Suspension medium.

A culture of ampicillin resistant *E. coli* was grown to saturation in Lambda Broth (i.e., 10 g/l tryptone, 0.042 M sodium chloride), 0.2% maltose, and 10 mM magnesium sulfate (Current Protocols in Molecular Biology, page 1.11.1).

To induce infection, 100 µl of the phage sample was added to 300 µl of the *E. coli* culture prepared above, and the culture was incubated for 10 minutes at 37°C. 2.5 ml Lambda Top Agar (i.e., 10.0 g/l tryptone, 42 mM sodium chloride, 7 g/l agar) was added to each phage-*E. coli* mixture, vortexed, and immediately spread onto Lambda plates (i.e., 10 g/l tryptone, 0.042 M sodium chloride, 10.0 g/l agar in 90 mm petri dishes).

After incubation of the plates for 16 hours at 37°C, lambda plaques were either counted, or, for plates with high confluence, the total surface area covered by plaques was estimated. The plaque forming units per milliliter (pfu/ml) were calculated by multiplying the dilution factor by the number of plaques appearing on each plate.

The plaque forming activity of Lambda phage was reduced by 5 orders of magnitude by alternating hydrostatic pressure at -10°C. The density of the frozen, pressure-cycled sample was found to be 9.4 x 10⁵ pfu/ml, while that of the frozen, unpressurized control was 3.2 x 10¹¹ pfu/ml. In a second experiment, the plaque forming activity was reduced from 9 x 10¹⁰ pfu/ml to 6.4 x 10⁴ pfu/ml after alternating pressure treatment. In contrast to the frozen samples, there was only a 3-fold decrease in plaque forming activity in the room temperature samples. The sample held at 36,000 psi for 5 minutes at 23°C yielded 1.2 x 10¹¹ pfu, and the control maintained at 14.7 psi had 3.1 x 10¹¹ pfu.

Serum had very little if any effect on plaque forming activity. There were 50 x 10¹¹ pfu in the sample diluted in Suspension medium, and the sample diluted 100-fold into serum decreased only to 3.1 x 10¹¹ pfu. Freezing just once at atmospheric pressure (14.7 psi) apparently had no effect on plaque forming activity. There were 3.2 x 10¹¹ pfu in the frozen sample and 3.1 x 10¹¹ pfu in the sample incubated at room temperature (23°C).

### Example 2: Effect of pulsation frequency on viral inactivation.

An experiment was carried out to determine the effect of pulsation frequency on the inactivation of lambda bacteriophage in serum.

A serum sample was inoculated with the virus and treated as in Example 1, but with varying frequencies of pulsation and a maximum pressure of 40,000 psi [275.8 MPa]. All samples were treated at -6°C, the total time of treatment for all samples was 15 minutes, and the time spent at high and low pressures was 7.5 minutes in each experiment. The experiment was carried out twice for each set of experimental conditions. The viral titers were measured as in Example 1.

A parallel experiment was carried out on a model of a therapeutic protein to see if its activity was maintained. Anti fluorescein goat IgG (Chemicon International; Tecuma, CA) was prepared to a concentration of 4 mg/ml in 5% glucose and 0.3% NaCl. The solution was then subjected to the same treatment as in the bacteriophage inactivation experiment and assayed by measurement of the ability to quench the fluorescence of a 50 nM solution of fluorescein. The following data were obtained:

| Frequency (cycles per hour) | phage titer (trial #1) | Trial #2 | % IgG activity |
|---|---|---|---|
| 0 | 5.0 x 10⁸ | 1.1 x 10⁷ | 100 |
| 4 | 4.6 x 10⁴ | 9.9 x 10² | 94 |
| 12 | 3.0 x 10³ | 7.7 x 10² | 97 |
| 20 | 3.4 x 10² | 5.0 x 10¹ | 92 |
| 40 | 4.8 x 10² | 0 | 95 |
| 60 | 7.0 x 10¹ | 0 | 100 |

This experiment demonstrates that the pulsation of pressure at low temperature can have a significant effect on the rate of viral inactivation, and can be useful in the production of therapeutic and diagnostic blood products. The process can operate under conditions of pressure and temperature that are consistent with high recovery of properly folded therapeutic proteins and can be effective against many types of viruses.

### Example 3: Acceleration of Nuclease Activity in a Hyperbaric Sterilization Process

Adult bovine serum is diluted to 50% (v/v) with water and chilled to 0°C. Four aliquots of the serum are dispensed into 250 µl microcentrifuge tubes such that there is 25 µl of air in each tube. The tubes are kept on ice until use. 2 µg of pUC19 plasmid DNA and 2 µg of yeast total RNA (Sigma) in 5 µl of 50 mM Tris buffer, pH 8.0, are added to each sample at the appropriate time. 5 µl of water is added to sample #4 before pressurization. The reactions are stopped by adding of 10 mM vanadyl ribonucleoside complexes and placing the samples on ice. The treatment of these samples are as follows:
Sample #1: control sample (i.e., nucleic acids only). In this sample, the vanadylribonucleosides are added prior to the nucleic acids.
Sample #2: serum and nucleic acid mixture, incubated at 25°C for 10 minutes. The reaction is immediately stopped as described above.
Sample #3: serum and nucleic acid mixture, pressurized to 60,000 psi at 25°C for 10 minutes. The reaction is stopped as described above.
Sample #4: serum, pressurized to 60,000 psi at 25°C for 10 minutes; the nucleic acids are then added, the mixture is incubated for 10 minutes, and the reaction is stopped as described above.

All samples are extracted with an equal volume of phenol/chloroform/isoamyl alcohol mixture to remove proteins, followed by precipitation of the nucleic acids in 70% ethanol. The nucleic acid pellet is re-dissolved in 20 µl of TE buffer. 10 µl of the resulting solution is loaded on a 0.8% agarose gel for electrophoresis. After electrophoresis, DNA is visualized by ethidium bromide fluorescence. 5 µl of the remaining sample is quantified using PicoGreen and Ribogreen dyes (Molecular Probes).

Samples #3 and #4 both show increased degradation with respect to sample #2. The nucleic acids in sample #4 are more degraded than the nucleic acids in sample #3. These results demonstrate that pressure can accelerate blood nucleases both by directly stimulating activity and by releasing inhibitors.

### Example 4: Pressure- and Temperature-Induced Inactivation of Saccharomyces cerevisiae

*Saccharomyces cerevisiae* was grown in YPD liquid medium (1% yeast extract, 2% peptone, 2% dextrose) until the cultures reached a density of 2 x 10⁶ cells/ml. The *S. cerevisiae* samples were then diluted 1:10 in calf serum and then subjected to various pressures and temperatures.

First, the temperature was held constant at 23.8°C during a pressurization process. One sample was maintained at 36,000 psi [248.4 MPa] for 10 minutes at 23.8°C. A second sample was cycled between 36,000 psi [248.4 MPa] and 14.7 psi [0,101 MPa] at 30 second intervals for 10 minutes at 23.8°C. The pressure and temperature were held constant at 14.7 psi [0,101 MPa] and 23.8°C, respectively, for a third sample as a positive control.

For the next three samples, temperature was reduced while equivalent pressure treatments were carried out. Thus, a fourth sample was cooled to -3.6°C and then pressurized to 36,000 psi [248.4 MPa] for 10 minutes. A fifth sample was also cooled to -3.6°C, then subjected to 10 cycles of pressure alternating between 36,000 psi [248.4 MPa] and 14.7 psi [0,101 mPa] at 30 sec intervals. As a positive control, a sixth sample was cooled to -3.6°C, but was not subjected to pressure treatment.

After the requisite pressure and temperature treatments, all of the samples were diluted by factors of 10, 100, and 1000, spread on LPD plates (1% yeast extract, 2% peptone, 2% dextrose, 1.5% agar), and grown at 32°C for 24 hours. The number of colony forming units (cfu) were calculated by multiplying the number of colonies by the dilution factor.

A pronounced inactivation of *Saccharomyces cerevisiae* by pressure treatments was observed at both 23.8°C and -3.6°C. The colony forming activity of the pressurized sample ranged from 1 X 10² cfu to 5.6 X 10³ cfu for the pressurized samples. The positive controls ranged in colony forming activity from 1.1 x 10⁵ cfu to 9.2 x 10⁵ cfu. Thus, the colony forming ability of *saccharomyces cerevisiae* was decreased by approximately 2 to 3 orders of magnitude.

### Example 5: Pressure and Temperature Induced Inactivation of the Moloney Murine Leukemia Virus

A retroviral vector, pLNCX, containing Phi+ (the extended viral packaging signal) and a neomycin resistance marker (Neo^{r}), but lacking viral structural genes, was used in conjunction with a packaging cell line, NIH-Cyt2. The NIH-Cyt2 cells express the gag, pol, and env viral structural genes necessary for particle formation and replication, but not the RNA packaging signal Phi+. Thus, the pLNCX vector and NIH-Cyt2 cell line together produce infectious, replication-incompetent particles structurally identical to the Moloney Murine Leukemia Virus (MMLV). These infectious MMLV particles contain RNA encoding the pLNCX vector.

MMLV infectious particles, suspended in DMEM with 10% calf serum (CS), are maintained at 4°C and 14.7 psi except during the pressure and temperature treatments described below. Hydrostatic pressure for one sample is cycled between 35,000 psi [241.5 MPa] and 14.7 psi [0,1 MPa] at 30 second intervals while the temperature is maintained at 2°C. As a positive control, the temperature of a second sample is maintained at 2°C for 10 minutes at 14.7 psi [0,1 MPa]. A third sample is cycled between 35,000 psi [241.5 MPa] and 14.7 psi [0,1 MPa] at 30 second intervals at -10°C. A fourth sample is held at 35,000 psi [241.5 MPa] for 5 minutes at -10°C. As a positive control, a fifth sample is frozen in dry ice and then warmed to -10°C for 5 minutes.

Samples are added to dishes of NIH-Cyt2 cells and incubated at 37°C for 30 minutes to transfect cells with the retroviral vector pLNCX. As a negative control, one plate of cells is mock-transfected with viral-free DMEM-CS. The cells are grown in DMEM with 10% calf serum and G418 which only allows cells with Neo^{r} to grow and thus selects for stable transformants. After a 10 day period, the dishes are rinsed with PBS, stained with methylene blue, rinsed again with PBS, and then the colonies are counted. The viral titer, expressed in colony forming units (cfu), is calculated by multiplying the dilution factor by the number of colonies.

### Example 6: Pressure-cycling inactivation of Escherichia coli-inoculated serum

A culture of ampicillin resistant *E. coli* strain in LB/amp media (Luria broth containing 50 µg/ml ampicillin) was grown to saturation. A sample of adult bovine serum (Sigma) was inoculated with 30 µl *E. coli* solution per ml serum. Aliquots (280 µl each) of the inoculated serum were placed into nine microcentrifuge tubes. The samples in the nine tubes were subjected to the following experimental conditions:
Sample 1 was left untreated as a control.
Sample 2 was maintained at atmospheric pressure (i.e, about 14.7 psi [0,101 MPa]) as the temperature was cycled twenty times between about -17°C and ambient temperature (i.e., about 25°C) over 20 minutes.
Samples 3-6 were cooled to -15°C at atmospheric pressure. The samples sat for 2 minutes to ensure thermal equilibrium. The samples were then subjected to pressure cycles at a rate of about 30 sec/cycle, or 2 Hz. Sample 3 was cycled ten times between about 14.7 psi [0,101 MPa] and about 15,000 psi [103,5 MPa] over 5 minutes; sample 4 was cycled ten times between about 14.7 psi [0,1 MPa] and about 35,000 psi [241.5 MPa] over 5 minutes; Samples 5 and 6 were cycled twenty times between about 14.7 [0,1 MPa] and about 35,000 psi [241.5 MPa] over 10 minutes.
Sample 7 was cycled twenty times between about 14.7 psi [0,1 MPa] and about 35,000 [241.5 MPa] psi at ambient temperature over 6 minutes, forty seconds.
Samples 8 and 9 were subjected to static pressurization for 2.5 minutes; sample 8 was pressurized at ambient temperature and sample 9 was pressurized at -15°C.

Dilutions of the samples were made in LB/amp media, and the samples were plated. Colony forming units per milliliter (CFU/ml) are shown below, along with log reductions (i.e., relative to the control):

| Sample # | CFU/ml | Log reduction |
|---|---|---|
| 1 | 1 x 10⁶ | 0 (control) |
| 2 | 3 x 10⁵ | 0.5 |
| 3 | < 1 x 10⁶ | > 0 |
| 4 | 8 x 10⁴ | 1.1 |
| 5 | 1 x 10⁴ | 2.0 |
| 6 | 6.4 x 10⁴ | 1.2 |
| 7 | 80 | 4.1 |
| 8 | 9.6 x 10³ | 2.0 |
| 9 | 4 x 10⁴ | 1.4 |

Thus, the most effective treatment was that corresponding to sample #7, cycling to 35,000 psi at about 25°C. The results indicate that cycling was more effective than static pressurization at the same temperature (cf. sample #8).

### Example 7: Sterilization of E. coli-contaminated needles

Two 20G needles were clipped, leaving 3 mm of the metal shaft attached to the plastic mounts. Tubes were constructed by cutting the ends off the plastic shaft of a 3 ml syringe and plugging each end with the rubber portions from two plungers. One ml of an overnight culture of *E*. *coli* was passed through each needle. Each needle was placed in a tube with 1 ml luria broth (LB) medium. There was approximately 0.2 ml of air left at the top of each tube.

One tube was subjected to 10 cycles of pressure treatment (each cycle including 30 seconds at 37,000 psi [255.3 MPa], followed by 30 seconds at 14.7 psi [0,101 MPa]) at 22.2°C. The second tube was placed inside the pressure chamber for 10 minutes at 22.2°C but was not subjected to pressure treatment.

After pressure treatment, the needles were removed from the tubes and 0.2 ml LB was passed through each needle. Half of the 0.2 ml LB was spread on an LB plate. The remaining 0.1 ml LB was diluted (1:10, 1:100, 1:1,000, ad 1:10,000) and spread on four LB plates. All plates were grown overnight at 37°C. Colonies were counted on each plate and the number of colony forming units (cfu) within each LB sample was calculated.

A dramatic difference was observed in the colony forming activity of the LB medium passed through the treated and untreated needles. There were 10 cfu in the 0.2 ml passed through the pressure treated needle, while there was 9.2 X 10⁵ cfu present in the LB passed through the untreated needle, nearly a 100,000-fold difference.

### Example 8: Pressure-shock sterilization

As a model for pathogenic viruses, fresh frozen plasma is spiked with 10⁸ plaque forming units (pfu) per ml of lambda bacteriophage stabilized with 10% glucose and 4 mM sodium caprylate. The plasma is placed in a high pressure vessel containing 50% ethylene glycol as a pressure transmitting medium and the temperature is equilibrated to -10°C. The pressure is increased to 150,000 psi over a period of 1 second and held at that pressure for an additional 1 second. The pressure is then released over the course of 2 seconds. The plasma sample is removed and dilutions are plated on lawns of E. *coli*. The plasma sample is found to be substantially free of infectious virus. The plasma proteins are analyzed by various methods including HPLC, IgG antigen binding, fluorescence enhancement of dansyl sarcosine by HSA, and clotting assays (including the activated partial thromboplastin time assay, APTT) to assess the integrity of the clotting factors.

### Example 9: Pressure-enhanced photochemical inactivation of viruses

A sample of bovine serum is inoculated with 10⁸ plaque forming units (pfu) per ml of lambda bacteriophage. 0.15 mM of psoralen is added. The sample is split into three aliquots. The three samples are treated as follows:
(1) no further treatment
(2) pressurization to 30,000 psi [206.8 MPa]
(3) pressurization to 30,000 psi [206.8 MPa] and simultaneous exposure to UVA light for 10 minutes.

All samples are held at a temperature of 25°C throughout the experiment. Treatment with pressure and light is accomplished by loading the sample into a quartz bottle with a polyethylene cap. The bottle is placed in an ethanol-filled high pressure spectroscopy cell (ISS, Champaign, IL) and pressurized. Samples are illuminated by aligning a window of the spectroscopy cell with a UVA lamp at a fixed distance. After treatment, the serum is serially diluted, mixed with *E*. *coli* and plated on agar. After overnight incubation at 37°C, the plaques on the plates are counted to arrive at the relative reduction of viral titer due to pressurization and the combination of pressurization and illumination. It is found that a significantly greater degree of viral inactivation is observed in sample #3, relative to samples #1 and #2. The experiment is repeated with lower concentrations of psoralen with the result that the combination of pressure and UVA light gives a rate of inactivation similar to that obtained by UVA alone, while resulting in less damage to therapeutic proteins. Similar experiments reveal that less light intensity or time of illumination is needed when the sample is pressurized. Experiments also show that nucleic-acid binding dyes that act in conjunction with oxygen (e.g. methylene blue) give results similar to those seen with psoralens.

### Example 10: Pressure enhanced chemical inactivation of viruses

A sample of bovine serum is inoculated with 10⁸ plaque forming units (pfu) per ml of lambda bacteriophage. The sample is split into four aliquots. The samples are treated as follows:
#1 no treatment
#2 0.1 mM iodine added and incubated for 10 minutes
#3 0.1 mM iodine added and pressurized to 30,000 psi for 10 minutes
#4 pressure of 30,000 psi [206.8 MPa] for 10 minutes

All samples are held at a temperature of 25°C throughout the experiment. After treatment, the reaction is quenched with a reducing agent and the serum is serially diluted, mixed with *E. coli* and plated on agar. After overnight incubation at 37°C, the plaques on the plates are counted to arrive at the relative reduction of viral titer due to pressurization and the combination of pressurization and chemical treatment. It is found that sample #3 has significantly greater reduction in viral titer (as compared to sample #1) than the sum of the reductions observed from samples #2 and #4, demonstrating a synergistic effect of pressure and iodine. Similar experiments are carried out with lower concentrations of chemical additives and it is found that pressure allows equivalent viral inactivation with lower concentrations of iodine or with shorter incubation time.

### Example 11: Effects of pressure cycling treatment on clotting time

Human plasma samples (BBI) were treated with 10, 2-minute pressure cycles at 40,000 psi [275.8 MPa] and temperatures ranging from -70°C to 20°C, and stored frozen at -70°C until use. The assay was initiated by addition of 60 µl plasma, 60 µl APTT reagent (Sigma) (37°C), and 60 µl of 25 mM CaCl₂ (37°C) to a 100 µl cuvette. The absorbance was observed at 550 nm and the clotting time was recorded as the time to an OD of 0.8 (approximately 1/2 maximum). It was found that the use of lower temperatures gave a dramatic improvement in clotting activity.

### Example 12: Integrity of IgG, IgM, HSA, and fX in cryobaric treatment

**IgG and IgM.** Commercial preparations (ACCURUN™, Boston Biomedica Inc.) of human plasma controls for Cytomegalovirus (CMV) antibody titer were treated with the pressure cycling technique and the ability of IgG and IgM to bind CMV antigen was measured using Abbott and Walpole CMV -antibody enzyme immunoassays. The assay comprises incubating the sample with polystyrene beads which are coated with heat inactivated CMV antigen. The beads are then washed and reacted with horseradish peroxidase labeled anti-human immunoglobulin. The beads are washed again and incubated with a chromogenic substrate which develops color at a rate indicative of the bound anti-CMV antibody. Dilutions of the ACCURUN material indicate that the assay is linear, suggesting that any loss of activity would have been detected. 260 µl samples of Accurun were equilibrated to -5°C and treated with 10 pressure cycles comprising 1 minute at elevated pressure and 1 minute at atmospheric pressure. Maximal pressures of about 10,000 [68.9 MPa] to about 50,000 psi [345 MPa] were used. Samples were removed and anti-CMV levels were measured. No significant loss of IgG or IgM activity was observed for pressures of up to about 50,000 psi [345 MPa].

**HSA.** Human serum albumin (HSA) integrity was determined by its ability to enhance the fluorescence of dansyl sarcosine. This assay is sensitive to changes in domain III of the protein and to overall levels of aggregation. Forty mg/ml of HSA was added to bovine serum which had been filtered with a microcon-30 spin-filter to remove any bovine albumin and larger proteins. The samples were subjected to pressure pulsation with a maximum pressure of about 34,000 psi [234.6 MPa] for 15 minutes with various frequency of pulsation at -5°C. Protein activity. was measured by successive addition of 10 µl volumes to a 3 ml solution of excess dansyl sarcosine in PBS. The results indicated that pressure pulsation is beneficial to protein integrity.

**Factor X**. To test the effect of the cryobaric process on an enzymatic blood factor, a chromogenic assay was performed on human ACD plasma treated at several pressures. The conditions used were identical to those in the IgG experiment: -5°C, 10 two-minutes cycles at about 19,000 psi [131.1 MPa] to about 50,000 psi [345 MPa]. Greater than 80% of factor X activity remained.

### Conclusions:

This data demonstrates that pressure cycling is capable of greater than 10⁷ reduction in titer of non-enveloped virus in a 15-minute period, yet IgG, IgM, factor X, and HSA activities can simultaneously be maintained at acceptable levels. Pulsation of pressure appears to enhance the retention of protein activity.

### Example 13: Pressure cycling inactivation of porcine parvovirus (PPV) in human plasma at very low temperature

Porcine Parvovirus (ATCC) is propagated in porcine testicle (PT) cells by infecting at a multiplicity of infection of 0.1 and incubating for 6 days. The cells are lysed by three freeze-thaw cycles and the solution is clarified by centrifugation. The resultant PPV titer is approximately 1 x 10¹⁰ infectious doses/ml.

Stock PPV is diluted 1:100 into human plasma and dispensed in 260 µl volumes into two flexible polyethylene microcentrifuge tubes (Cole Parmer). One tube is designated "control" and the other "experimental". The tubes are sealed with Dow Corning silicone high vacuum grease, and wrapped in PARAFILM® (American National Can, Menasha, WI).

The high pressure reaction chamber is filled with 70% ethanol and 0.2% rhodamine B as a tracer and cooled to -40°C. The experimental tube is placed in the high pressure reaction chamber for 6 minutes to equilibrate the temperature and the pressure is increased over a period of 5 seconds to a pressure of 80,000 psi [552 MPa]. The pressure is held for 10 minutes at 80,000 psi [552 MPa] and then released over a period of 2 seconds. The temperature is then allowed to equilibrate for 5 minutes. This process is repeated for a total of 20 cycles. The control sample is incubated for an equivalent time at -10°C.

The PPV titers of the control and experimental samples are determined by performing half-log serial dilutions in Hanks Buffered Saline Solution (HBSS). Each dilution is used to inoculate 6 wells of a 96 well culture plate containing 50% confluent PT cells.

After incubating the cells for 5 days in MEM media supplemented with 7.5% fetal bovine serum, the presence of virus is determined by fixing the plates in 80% acetone for 20 minutes and incubating each well with 50 µl of fluorescein-conjugated anti-PPV antibody (VMRD Inc., Pullman WA) for 40 minutes at 37°C, washing 3 times with water, and observing with an epifluorescence microscope. TCID50 is determined by the Spearman-Karber method. The experimental sample is found to have no detectable virus and the control sample has a titer of 10⁸ infectious doses/ml. Clotting time analysis is performed by a single stage activated partial thromboplastin time (APTT) measurement and the clotting time is found to be about 30 seconds for both the control and experimental samples indicating that the plasma is suitable for purification of blood products and transfusion.

### Example 14: Pressure cycling inactivation of bacteria and fungi

Various species of bacteria were subjected to pressure-cycling protocols to determine the rate of inactivation. Two-hundred and fifty microliter volumes of each species were aliquoted into Cole Palmer polyethylene tubes. The tubes were frozen at -70°C and a layer of high vacuum grease was added to the top of the frozen liquid layer. Each tube was then wrapped in a layer of PARAFILM® (American National Can, Menasha, WI).

The following bacterial species were tested:
*Bacillus cereus* ATCC 14579 (initial titer: 6.6 x 10⁸ cfu/ml), *Enterococcus faecium* ATCC 49032 (3.3 x 10⁸), two samples *Escherichia coli* ATCC 43894 (8.6 x 10⁹ and 5.4 x 10⁷), *Pseudomonas aeruginosa* ATCC 14502 at 9.5 x 10⁷, and *Staphylococcus* aureus ATCC 9144 at 9.9 x 10⁷.

Ten two-minute pressure cycles (i.e., 1 minute at ambient pressure, one minute at up to 50,000 psi [345 MPa]) were carried out at -10°C on each bacterial culture. Certain samples of each species were also subjected to 10 minutes of static pressure at both about 29,000 [200.1 MPa] and about 50,000 psi [345 MPa]. Each sample was equilibrated to the chamber temperature for five minutes prior to pressurization. The chamber was filled with 50% ethylene glycol in water and spiked with 1% rhodamine, to determine if any of the chamber liquid has leaked into the sample during pressurization. Following the pressure cycling procedure, the samples were placed at -70°C until assayed.

The samples were assayed to determine the titer (cfu/ml) following the pressurization process. Serial dilutions were made from an aliquot of each sample. Each dilution was plated onto 10 ml solid agar and incubated overnight at 37°C. The titer of each plate was counted to determine the rate of inactivation (final titer). The initial titer was divided by the final titer to determine the reduction in microbial number.

All gram positive and gram negative bacteria examined were inactivated. *Bacillus* cereus was inactivated by 5 logs, Enterococcus faecium by 6 logs, E. coli by 7 logs, *Pseudomonas* aeruginosa by 8 logs, *Staphylococcus* aureus by 6 logs and Candida albicans by 5 logs. For all bacteria, pressure pulsation was more effective than static pressure. The most dramatic difference was seen with *Enterococcus,* which was inactivated by less than 1 log without pressure cycling but by approximately 6 logs with pressure cycling at 50,000 psi [345 MPa]. *Pseudomonas* was inactivated by approximately 6 logs with pressure cycling and 2.5 logs with static pressure at around 29,000 psi [200,1 MPa]. The rate of *Pseudomonas* inactivation at 50,000 psi [345 MPa] with pressure cycling was greater than 8 logs, but with static pressure remained at 6 logs. The Staphylococcus sample was inactivated by 2 and 6 logs with pressure-cycling at 29,000 psi [200,1 MPa] and 50,000 psi [345 MPa] respectively, but only 1 and 3 logs with the same conditions under static pressure. The difference in the rate of inactivation of the low titer *E*. *coli* at 29,000 psi [200,1 MPa] was 67-fold with cycling and 6.4-fold with static pressure. The rate of inactivation at 50,000 psi [345 MPa] increased to 4 logs with cycling but only to 2 logs with static pressure. The high titer E. *coli* yielded an increase of 4 logs of inactivation with pressure cycling compared to static pressure at both 29,000 psi [300.1 MPa] and 50,000 psi [345 MPa].

Pressure pulsation thus yielded an increase in inactivation over static pressure for all species of bacteria tested, with the benefit of pressure-cycling being more pronounced at 50,000 psi [345 MPa] than at 29,000 psi [200.1 MPa] for all species.'

### Example 15: Inactivation of enveloped viruses

To test the efficacy of cryobaric treatment against enveloped viruses, Human Immunodeficiency Virus (HIV-1) and Herpes Simplex Virus (HSV-1) were treated and their infectivity measured. For the HIV experiment, the cryobaric treatment consisted of 5 two minute pressure cycles at -10°C. Each cycle consisted of 1 minute at high pressure and 1 minute at atmospheric pressure. An approximately 6 log reduction in HIV infectivity was achieved when the pressure was elevated to 50,000 psi [345 MPa]. Herpes Simplex 1 was also inactivated by 6 logs at this pressure. This treatment is compatible with retention of the integrity and activity of most therapeutic proteins, as shown, for example, in Example 21.

### Example 16: Inactivation of MS2: static pressure conditions

MS2-infected plasma samples were pressurized to 80,000 psi at -26°C, then the temperature was increased to -14°C for 5, 10, 15, 30 and 60 minutes. Two plasma samples were treated for each experiment, one with phage for inactivation studies and one without phage for factor analysis.

Following cryobaric treatment, MS2 infectivity was assayed according to the following protocol. Host *E*. *coli* were grown to saturation overnight in MS2 Broth (containing 10 g/l tryptone and 42 mM sodium chloride), supplemented with 0.2% maltose and 10 mM magnesium sulfate. Following dilution of treated phage, where necessary, 100 µl of phage sample was added to 100 µl of the *E*. *coli,* incubated at 23°C for 20 minutes, then incubated at 37°C for 10 minutes. The phage-*E*. *coli* mixture was added to 2.5 ml melted Top Agar (10 g/l tryptone, 42 mM sodium chloride, 7 g/l agar, 47°C), vortexed, and immediately spread onto 90 mm petri dishes containing 10 g/l tryptone, 42 mM sodium chloride, and 10 g/l agar. After incubation for approximately 16 hours at 37°C, plaques were counted.

The samples for factor VIII analysis were stored at -70°C until assay. Factor VIII assays were performed with the American Diagnostica chromagenic assay kit (Greenwich, CT).

The results were as follows, with "Inactivation" representing the titer (in pfu/ml) for the untreated sample divided by the titer for each given sample, and "fVIII" representing the percentage of factor VIII activity remaining for each sample after pressure treatment (i.e., relative to the untreated sample):

| Time(min) | Titer | Inactivation | fVIII |
|---|---|---|---|
| 0 | 3.2e9 | ---- | 100% (defined) |
| 5 | 7.5e6 | 4.3e2 | 95 |
| 10 | 7.0e6 | 4.6e2 | 79 |
| 15 | 5.4e6 | 5.9e2 | 87 |
| 30 | 6.0e6 | 5.3e2 | 72 |
| 60 | 1.3e6 | 2.6e3 | 67 |

These data show that approximately first order kinetics were observed for factor VIII loss, while MS2 inactivation was relatively independent of incubation time, suggesting that the viral inactivation was occurring in the initial pulse to 80,000 psi [552 MPa] at -26°C.

### Example 17: Inactivation of MS2: temperature optimization

In an experiment to determine the optimum temperature for MS2 inactivation, the conditions indicated in Example 16 were employed with the exception that each sample was equilibrated for 5 minutes to the corresponding starting temperature indicated in the data table below. The samples were pressurized to approximately 80,000 psi [552 MPa] for 2 minutes, followed by depressurization and temperature equilibration for 5 minutes. The pressurization cycle was repeated 2 more times. The results were as follows:

| Temperature | Titer | Inactivation | fVIII |
|---|---|---|---|
| -20°C | 3.2e6 | 3.4 | 47% |
| -17°C | 4.6e5 | 4.2 | 36% |
| -14°C | 1.2e4 | 5.8 | 9% |
| -11°C | 5.0e3 | 6.2 | 0% |
| -11°C | 7.4e9 | | 100% (control) |

These data demonstrate significant (i.e., 3 log) inactivation of MS2 under conditions (-20°C) that give 50% recovery of fVIII. The higher temperatures increased inactivation of MS2 but decreased recovery of fVIII.

### Example 18: Inactivation of MS2: pressure optimization

In an experiment to determine the optimum pressure for MS2 inactivation, the conditions indicated in Example 16 were employed with the exceptions that each sample was equilibrated to -17°C for 5 minutes and the samples were pressurized to between 60,000 and 80,000 psi [414 MPa and 552 MPa], as indicated below. The indicated pressures were held for 60 seconds, followed by depressurization and temperature equilibration for 5 minutes. The time of pressurization (about 3 seconds) and depressurization (about 1 second) are not included in the reported pressurization time. The results were as follows:

| Pressure (psi) | Titer | Log inactivation | fVIII | %loss/log |
|---|---|---|---|---|
| 60,000 [414 MPa] | 2.6e9 | 0.3 | 53 | 157 |
| 65,000 [448,5 MPa] | 1.3e9 | 0.6 | 45 | 91 |
| 70,000 [482,6 MPa] | 7.0e7 | 1.9 | 47 | 28 |
| 75,000 [517,5 MPa] | 9.4e5 | 3.8 | 47 | 14 |
| 80,000 [552 MPa] | 1.1e6 | 3.7 | 32 | 18 |
| control | 5.8e9 | | | |

The data indicate that the optimum pressure under these temperature and timing conditions was about 75,000 psi.

### Example 19: Inactivation of MS2: pulsation timing optimization

To measure the relative rates of MS2 inactivation and loss of fVIII activity as a function of the time at elevated pressure for each pulse, conditions similar to those employed in Example 18 were used. Each sample was equilibrated to -20°C for 5 minutes. The samples were pressurized to approximately 80,000 psi [552 MPa] and held there for between 15 and 120 seconds, as indicated below, followed by depressurization and temperature equilibration for 5 minutes. The pressurization cycle was repeated 2 more times. The times required for pressurization (i.e., about 3 seconds) and depressurization (i.e., about 1 second) are not included in the reported pressurization time. A second round of experiments was conducted with pressure pulse durations of 10 to 120 seconds. The results of the two experiments were as follows:

| Time (sec) | Titer | log inactivation | fVIII | loss/log |
|---|---|---|---|---|
| 15 | 6.4e5 | 4.0 | 73 | 6.5 |
| 30 | 7.2e6 | 2.9 | 65 | 12 |
| 60 | 1.2e6 | 3.7 | 48 | 14 |
| 120 | 3.0e6 | 3.3 | 51 | 15 |
| 240 | 1.5e6 | 3.6 | 44 | 15 |
| control | 5.8e9 | | | |
| | | | | |
| 10 | | 3.3 | 72 | 8.5 |
| 15 | | 3.7 | 65 | 9.4 |
| 30 | | 4.0 | 44 | 14.0 |
| 120 | | 4.8 | 19 | 16.9 |

These data indicate that decreasing the pressure pulse duration to times as short as 10 seconds has a positive effect on the retention of fVIII while having little or no effect on the level of MS2 inactivation.

### Example 20: Inactivation of MS2: pH effect

Three milliliters of human plasma was spiked with 50 µl of MS2 stock in LB. A 10X stock solution of 3-[cyclohexylamino]-1-propane sulfonic acid (CAPS) was prepared and diluted 1:10 with spiked plasma. This sample and a plasma-only control sample were frozen at -70°C and sealed with silicone grease and PARAFILM®. Before treatment, each sample was placed into the reaction chamber for 4 minutes to equilibrate to the experimental temperature.

100 µl of each sample was removed for serial dilution. Phage were 100-fold serially diluted in lambda broth in microcentrifuge tubes. To ensure proper mixing, the tubes were vortexed before further dilution. 100 µl of each dilution was mixed with 100 µl of log phase MS2-host *E*. *coli,* incubated for 10 minutes at room temperature, and incubated for 20 minutes at 37°C. Three milliliters of lambda top agar (at 47-50°C) was added, then the tubes were briefly vortexed and poured over lambda agar plates. The samples were exposed to a pressure of 60,000 psi [414 MPa] for 10 minutes, then assayed.

The results were as follows:

| Condition | titer |
|---|---|
| 100 mM CAPS pH 10.5 | 3.0e6 |
| control | 3.9e9 |

The data indicate that addition of buffer to increase the pH allowed inactivation of the virus to occur at a pressure at which no activation occurred upon treatment of the neutral solution.

Example 21: Inactivation of pathogens in clinical specimens Samples of human plasma were equilibrated over a period of 4 minutes to -10°C. The pressure was increased to an elevated pressure of 20,000 psi [138 MPa] for 1 minute. The pressure was reduced to atmospheric pressure and equilibrated for 1 minute. This process was repeated 'nine more times. Similar treatments were performed using elevated pressures of 30,000 [206.8 MPa], 40,000 [275.8 MPa], 50,000 [345 MPa] and 60,000 psi [414 MPa]. A control sample was held at -10°C for an equivalent period of time (about 20 minutes), without increasing the pressure.

After treatment, clinical assays were performed to determine the activity of various analytes. The following analytes were unaffected by any of the pressure cycling treatments:
Uric acid
Calcium
Phosphorous
Glucose
Creatinine
Urea Nitrogen
Na, K, Cl, Mg, Bicarbonate
Triglycerides GB
Total Bilirubin
Total Protein
Albumin
Cholesterol
Lipase
Anion Gap
BUN/Creatinine Ratio
Albumin/Globulin Ratio
Alkaline phosphatase
The activities of the following multimeric enzymes were reduced in the samples exposed to pressures of 30,000 psi or more:
Creatine kinase (CK)
Alanine aminotransferase (ALT)
Aspartate aminotransferase (AST)
Liver alcohol dehydrogenase (LD)
Gamma glutamyl transferase (GGT)

Although there was enough residual activity remaining, for example, to measure such differences between normal and elevated levels of these enzymes as would occur in a patient with liver disfunction, a sterilization method that does not reduce the activity of multimeric enzymes was desired.

To this end, the following experiment was carried out: Human plasma samples were equilibrated to -10°C for 4 minutes and the pressure was elevated to 80,000 psi [652 MPa] over a period of about 5 seconds and held for a defined duration. The pressure was then lowered to ambient over about 2 seconds. The process was repeated 2 more times. The durations at elevated pressure tested were 1, 5, 10, 30, 60, 120, and 240 seconds. The plasma samples were then tested for enzymatic activity.

The activity of the two monomeric enzymes tested, lipase and amylase, were unaffected at any pressure duration.

With a 30 second or greater duration, the activities of LD, CK, AST, and ALT were almost completely eliminated while the activities of GGT and alkaline phosphatase (ALK) were reduced by about half. With a 5 second duration, all of the enzyme activities were improved. With a 1 second duration, ALT, GGT and ALK had near complete activity, and LD, AST and CK had 40% to 70% of the activity of the untreated control.

Even at the short durations, acceptable levels of inactivation of pathogens can be obtained, for example, in the preceding Examples.

## Claims

1. A method for sterilizing a material containing a microbe, the method comprising:
providing a material at an initial pressure and temperature;
cooling the material to a temperature Te, wherein Te is -40°C to 10°C;
exposing the material to an elevated hydrostatic pressure sufficient to inactivate at least some of the microbe in the material;
decreasing the pressure to a reduced pressure, and repeatedly cycling the pressure between the elevated pressure and the reduced pressure thereby providing a sterilized material.

2. The method of claim 1, wherein the material further comprises a protein, and the elevated pressure is insufficient to irreversibly denature the protein.

3. The method of claim 1, wherein the elevated pressure is sufficient to inactivate at least about 10% of the microbe in the material.

4. The method of claim 1, wherein the reduced pressure is about 1 atm.

5. The method of claim 1, wherein the initial temperature is about 25°C.

6. The method of claim 1, wherein the initial temperature is less than about 0°C.

7. The method of claim 6, wherein the initial temperature is between -20°C and -5°C.

8. The method of claim 1, wherein the elevated pressure is in the range of about 5,000 psi to about 95,000 psi [34.5 to about 655.0 MPa].

9. The method of claim 1, wherein the elevated pressure is in the range of about 30,000 to about 75,000 psi [206.8 to about 517.1 MPa].

10. The method of claim 1, wherein the elevated pressure is in the range of about 95,000 to about 150,000 psi [655.0 to about 1034.2 MPa].

11. The method of claim 1, further comprising cooling the material to a decreased temperature prior to the pressure increasing and cycling steps.

12. The method of claim 11, wherein said decreased temperature is between about -40° and about 0°C.

13. The method of claim 11, wherein the decreased temperature is, between about -20° and about -5°C.

14. The method of claim 1, wherein the material is a suspension of pathogenic cells.

15. The method of claim 14, further comprising, removing from the suspension substantially all of any toxins that may be present in the suspension.

16. The method of claim 1, wherein the initial temperature is between -40°C and 0°C.

17. The method of claim 1, further comprising providing sufficient time to allow the temperature of the material to equilibrate prior to each increasing of the pressrure.

18. The method of claim 1, further comprising cooling the material.

19. The method cf claim 18, wherein the cooling step is carried out prior to the pressure increasing step.

20. The method or claim 18, wherein the cooling step is carried out after the pressure increasing step but before the pressure decreasing step.

21. The method of claim 18, wherein the material is cooled to a temperature in the range from about -40° to about 0°C.

22. The method of claim 18, wherein the material is cooled to a temperature in the range from about -20° to about -5°C.

23. The method of any one of claims 18-22, further comprising warming the material to an increased temperature before the pressure decreasing step.

24. The method of any one of claims 18-22, further comprising warming the material to an increased temperature after the pressure decreasing step.

25. The method of claim 1, further comprising, prior to the pressure increasing step, adjusting the pH of the material to a pH greater than about 10.

26. The method of claim 1, further comprising, prior to the pressure increasing step, adjusting the pH of the material to a pH less than about 4.

27. The method of claim 1, wherein the material being sterilized is selected from the group consisting of a biological sample, blood plasma, therapeutic and diagnostic products derived from blood plasma, biological fluids, medical fluids, medicaments, research solutions and reagents, serum, living tissue, medical or military equipment, a foodstuff, a pharmaceutical preparation, and a vaccine.

28. The method of claim 1, wherein the microbe comprises one or more members of the group consisting of a bacterium, a virus, a fungus, a protist, a spore former, protozoan parasites, helminth parasites, malaria-inducing organisms, giardia, and a virally infected cell.

29. The method of claim 1, wherein a phase-change catalyst is added to the material prior to sterilization.

30. The method of claim 1, wherein a protein-stabilizing agent is added to the material prior to sterilization.

31. The method of claim 30, wherein the protein-stabilizing agent is selected from the group consisting of sugars, glycerol, a hydrophilic polymer, a cyclodextrin, a caprylate, acetyl tryptophanoate, polyethylene glycol, anti-oxidant, and a protein specific ligand.

32. The method of claim 1, wherein a nucleic acid-binding compound is added to the material prior to sterilization.

33. The method of claim 32, wherein the nucleic acid-binding compound is a photosensitizer.

34. The method of claim 33, wherein the photosensitizer is a psoralan.

35. The method of claim 1, wherein the material to be sterilized is provided in its final packaging, the packaging being adapted to transmit pressure without rupture.

36. The method of claim 35, wherein the packaging is hermetically sealed in flexible plastic.

37. The method of claim 35, wherein the packaging is a syringe and pressure is transmitted via a plunger.

38. The method of claim 1, wherein the providing step comprises charging the material into a container adapted to transmit an external pressure to the material; and submerging the container in a pressurizing medium prior to increasing the pressure.

39. The method of claim 38, wherein the pressurizing medium is a sterilizing solution selected from the group consisting of an oxidizing agent, an alcohol, urea, à guanidinium salt, an acid, and a base.

40. The method of claim 1, wherein the microbe is a virus.

41. The method of claim 1, wherein
the material includes a protein, and
the elevated pressure is sufficient to irreversibly denature substantially all of the protein if the elevated pressure is maintained for a time substantially longer than tₑ, but wherein the elevated pressure is insufficient to irreversibly denature the protein when the elevated pressure is maintained for time period tₑ or less.

42. The method of claim 41, wherein the elevated pressure is sufficient to denature substantially all of the protein if the elevated pressure is maintained for a time period longer than ten times tₑ.

43. The method of claim 41, wherein the elevated pressure is sufficient to denature substantially all of the protein if the elevated pressure is maintained for a time period longer than three times tₑ.

44. The method of claim 41, wherein the protein comprises one or more blood clotting factors.

45. The method of claim 41, wherein the protein comprises one or more immunoglobulins.

46. The method of claim 41, wherein the protein comprises one or more monomeric proteins.

47. The method of claim 41, wherein the protein comprises one or more multimeric proteins.

48. The method of claim 44, wherein the material is selected from the group consisting of blood plasma, therapeutic and diagnostic products derived from blood plasma, biological fluids, medical fluids, medicaments, research solutions, living tissue, and pharmaceutical preparations.

49. The method of claim 40, wherein the virus comprises a non-encapsulated virus.

50. The method of claim 40, wherein the virus is selected from the group consisting of human parvovirus B19, porcine parvovirus, bovine parvovirus, human immunodeficiency virus, herpes simplex virus, hepatitis A virus, and transfusion transmitted virus.

51. The method of claim 41, wherein the elevated pressure is 10,000 to 120,000 psi [68.9 to 827.4 MPa].

52. The method of claim 41, wherein the elevated pressure is 40,000 to 100,000 psi [275.8 to 689.5 MPa].

53. The method of claim 41, wherein the elevated pressure is 70,000 psi to 90,000 psi [482.6 to 620.5 MPa].

54. The method of claim 1, wherein the reduced pressure, is intermediate between the initial pressure and the elevated pressure.

55. The method of claim 41, wherein time tₑ is 0.5 to 300 seconds.

56. The method of claim 55, wherein time tₑ is 10 to 30 seconds.

57. The method of claim 1, wherein the material is exposed to between 2 and 100 cycles.

58. The method of claim 1, wherein the material is exposed to at least about 3 cycles.

59. The method of claim 1, wherein the material is exposed to at least about 10 cycles.

60. The method of claim 1, wherein the material is exposed to at least about 100 cycles.

61. Producing a vaccine by inactivating the virus of a suspension of a virus by the method of Claim 40.

## Patentansprüche

1. Ein Verfahren zur Sterilisierung eines Materials, das eine Mikrobe enthält, wobei das Verfahren umfasst:
zur Verfügung stellen eines Materials bei einem Anfangsdruck und Temperatur;
Kühlen des Materials auf eine Temperatur Te, wobei Te -40°C bis 10°C ist;
Aussetzen des Materials einem erhöhten hydrostatischen Druck, der ausreicht, um wenigstens einige der Mikroben in dem Material zu inaktivieren;
Verringern des Drucks auf einen reduzierten Druck und wiederholtes zyklisches Durchlaufen des Drucks zwischen einem erhöhten Druck und einem reduzierten Druck,
dabei zur Verfügung stellen eines sterilisierten Materials.

2. Verfahren nach Anspruch 1, wobei das Material weiterhin ein Protein umfasst und der erhöhte Druck nicht ausreicht, um das Protein irreversibel zu denaturieren.

3. Verfahren nach Anspruch 1, wobei der erhöhte Druck ausreichend ist, um wenigstens 10% der Mikroben in dem Material zu inaktivieren.

4. Verfahren nach Anspruch 1, wobei der reduzierte Druck etwa 1 atm ist.

5. Verfahren nach Anspruch 1, wobei die Anfangstemperatur etwa 25°C ist.

6. Verfahren nach Anspruch 1, wobei die Anfangstemperatur weniger als etwa 0°C ist.

7. Verfahren nach Anspruch 6, wobei die Anfangstemperatur zwischen -20°C und -5°C ist.

8. Verfahren nach Anspruch 1, wobei der erhöhte Druck in einem Bereich von etwa 5000 psi bis etwa 95000 psi [34,5 bis etwa 655,0 MPa] ist.

9. Verfahren nach Anspruch 1, wobei der erhöhte Druck in einem Bereich von etwa 30000 bis etwa 75000 psi [206,8 bis etwa 517,1 MPa] ist.

10. Verfahren nach Anspruch 1, wobei der erhöhte Druck in einem Bereich von etwa 95000 bis etwa 150000 psi [655,0 bis etwa 1034,2 MPa] ist.

11. Verfahren nach Anspruch 1, weiterhin umfassend, Kühlen des Materials auf eine verringerte Temperatur vor der Erhöhung des Drucks und den zyklischen Schritten.

12. Verfahren nach Anspruch 11, wobei die verringerte Temperatur zwischen etwa -40°C und etwa 0°C ist.

13. Verfahren nach Anspruch 11, wobei die verringerte Temperatur zwischen etwa -20°C und etwa -5°C ist.

14. Verfahren nach Anspruch 1, wobei das Material eine Suspension pathogener Zellen ist.

15. Verfahren nach Anspruch 14, weiterhin umfassend, Entfernen im Wesentlichen aller Toxine, die in der Suspension sein können, aus der Suspension.

16. Verfahren nach Anspruch 1, wobei die Anfangstemperatur zwischen -40°C und 0°C ist.

17. Verfahren nach Anspruch 1, weiterhin umfassend, zur Verfügung stellen von ausreichend Zeit, um es der Temperatur des Materials zu ermöglichen vor jedem Steigern. des Drucks zu equilibrieren.

18. Verfahren nach Anspruch 1, weiterhin umfassend, Kühlen des Materials.

19. Verfahren nach Anspruch 18, wobei der Kühlschritt vor dem Drucksteigerungs-Schritt durchgeführt wird.

20. Verfahren nach Anspruch 18, wobei der Kühlschritt nach dem Drucksteigerungs-Schritt, jedoch vor dem Druckverringerungs-Schritt durchgeführt wird.

21. Verfahren nach Anspruch 18, wobei das Material auf eine Temperatur im Bereich von etwa -40°C bis etwa 0°C gekühlt wird.

22. Verfahren nach Anspruch 18, wobei das Material auf eine Temperatur im Bereich von etwa -20°C bis etwa -5°C gekühlt wird.

23. Verfahren nach einem der Ansprüche 18-22, weiterhin umfassend, Erwärmen des Materials auf eine gesteigerte Temperatur vor dem Druckverringerungs-Schritt.

24. Verfahren nach einem der Ansprüche 18-22, weiterhin umfassend, Erwärmen des Materials auf eine gesteigerte Temperatur nach dem Druckverringerungs-Schritt.

25. Verfahren nach Anspruch 1, weiterhin umfassend, Einstellen des pH des Materials vor dem Drucksteigerungs-Schritt auf einen pH größer als etwa 10.

26. Verfahren nach Anspruch 1, weiterhin umfassend, Einstellen des pH des Materials vor dem Drucksteigerungs-Schritt auf einen pH geringer als etwa 4.

27. Verfahren nach Anspruch 1, wobei das sterilisierte Material ausgewählt ist aus der Gruppe bestehend aus, einer biologischen Probe, Blutplasma, therapeutischen oder diagnostischen Produkten, die aus Blutplasma stammen, biologischen Flüssigkeiten, medizinischen Flüssigkeiten, Medikamenten, Forschungslösungen und Reagenzien, Serum, lebendem Gewebe, medizinischer oder militärischer Ausstattung, einem Nahrungsmittel, einer pharmazeutischen Zubereitung und einem Impfstoff.

28. Verfahren nach Anspruch 1, wobei die Mikrobe ein oder mehrer Mitglieder der Gruppe bestehend aus, einem Bakterium, einem Virus, einem Pilz, einem Protisten, einem Sporenbildner, protozoische Parasiten, Darmwurm Parasiten, malaria-induzierende Organismen, Giardia und einer viral infizierten Zelle, umfasst.

29. Verfahren nach Anspruch 1, wobei ein Phasenübergangs-Katalysator dem Material vor der Sterilisierung zugesetzt wird.

30. Verfahren nach Anspruch 1, wobei ein Proteinstabilisierungs-Mittel dem Material vor der Sterilisierung zugesetzt wird.

31. Verfahren nach Anspruch 30, wobei das Proteinstabilisierungs-Mittel ausgewählt ist, aus der Gruppe bestehend aus Zuckern, Glyzerin, einem hydrophilen Polymer, einem Cyclodextrin, einem Caprylat, Acetyl-Tryptophanoat, Polyethylen-Glykol, Antioxidationsmittel, und einem protein-spezifischen Liganden.

32. Verfahren nach Anspruch 1, wobei dem Material eine Nukleinsäure-bindende Verbindung vor der Sterilisierung zugesetzt wird.

33. Verfahren nach Anspruch 32, wobei die Nukleinsäure-bindende Verbindung ein Photosensibilisator ist.

34. Verfahren nach Anspruch 33, wobei der Photosensibilisator ein Psoralen ist.

35. Verfahren nach Anspruch 1, wobei das zu sterilisierende Material in seiner Endverpackung zur Verfügung gestellt wird, und die Verpackung so angepasst ist, das sie den Druck ohne zerreißen überträgt.

36. Verfahren nach Anspruch 35, wobei die Verpackung hermetisch in flexiblem Plastik verschlossen ist.

37. Verfahren nach Anspruch 35, wobei die Verpackung eine Spritze ist und der Druck über einen Kolben übertragen wird.

38. Verfahren nach Anspruch 1, wobei der Schritt der zur Verfügungsstellung umfasst, Einfüllen des Materials in einen Behälter, der angepasst ist, um einen externen Druck auf das Material zu übertragen; und
Untertauchen des Behälters in einem Druckmittel, bevor der Druck erhöht wird.

39. Verfahren nach Anspruch 38, wobei das Druckmittel eins Sterilisierungs-Lösung, ausgewählt aus der Gruppe basisband aus, einem Oxidationsmittel, einem Alkohol, Harnstoff, einem Guanidinium Salz, einer Säure und einer Base, ist.

40. Verfahren nach Anspruch 1, wobei die Mikrobe ein Virus ist.

41. Verfahren nach Anspruch 1, wobei das Material ein Protein einschließt und der erhöhte Druck ausreichend ist, um im Wesentlichen alle Proteine irreversibel zu denaturieren, wenn der erhöhte Druck für eine Zeit, wesentlich länger als tₑ aufrechterhalten wird, aber wobei der erhöhte Druck nicht ausreichend ist, um das Protein irreversible zu denaturieren, wenn der erhöhte Druck für einen Zeitraum von tₑ oder weniger aufrecht erhalten wird.

42. Verfahren nach Anspruch 41, wobei der erhöhte Druck ausreichend ist, um im Wesentlichen alle Proteine zu denaturieren, wenn der erhöhte Druck für einen Zeitraum von länger als zehnmal tₑ aufrechterhalten wird.

43. Verfahren nach Anspruch 41, wobei der erhöhte Druck ausreichend ist, um im Wesentlichen alle Proteine zu denaturieren, wenn der erhöhte Druck für einen Zeitraum von länger als dreimal tₑ aufrechterhalten wird.

44. Verfahren nach Anspruch 41, wobei das Protein einen oder mehrere Blutgerinnungsfaktoren umfasst.

45. Verfahren nach Anspruch 41, wobei das Protein ein oder mehrere Immunglobuline umfasst.

46. Verfahren nach Anspruch 41, wobei das Protein ein oder mehrere monomere Proteine umfasst.

47. Verfahren nach Anspruch 41, wobei das Protein ein oder mehrere multimere Proteine umfasst.

48. Verfahren nach Anspruch 41, wobei das Material ausgewählt ist, aus der Gruppe bestehend aus, Blutplasma, therapeutischen oder diagnostischen Produkten, die aus Blutplasma stammen, biologischen Flüssigkeiten, medizinischen Flüssigkeiten, Medikamenten, Forschungslösungen, lebendem Gewebe und pharmazeutischen Zubereitungen.

49. Verfahren nach Anspruch 40, wobei das Virus ein nicht-verkapseltes Virus umfasst.

50. Verfahren nach Anspruch 40, wobei das Virus ausgewählt ist, aus der Gruppe bestehend aus, humanem Parvovirus B19, Schweine-Parvovirus, Rinder-Parvovirus, humanem Immundefizienz Virus, Herpes Simplex Virus, Hepatitis A Virus und transfusionsvermitteltem Virus.

51. Verfahren nach Anspruch 41, wobei der erhöhte Druck 10000 bis 120000 psi [68.9 bis 827,4 MPa] ist.

52. Verfahren nach Anspruch 41, wobei der erhöhte Druck 40000 bis 100000 psi [275,8 bis 689,5 MPa] ist.

53. Verfahren nach Anspruch 41, wobei der erhöhte Druck 70000 bis 90000 psi [482,6 bis 620,5 MPa] ist.

54. Verfahren nach Anspruch 1, wobei der reduzierte Druck zwischen dem Anfangsdruck und dem erhöhten Druck liegt.

55. Verfahren nach Anspruch 41, wobei die Zeit tₑ 0,5 bis 300 Sekunden ist.

56. Verfahren nach Anspruch 55, wobei die Zeit tₑ 10 bis 30 Sekunden ist.

57. Verfahren nach Anspruch 1, wobei das Material zwischen 2 und 100 Zyklen ausgesetzt wird.

58. Verfahren nach Anspruch 1, wobei das Material wenigstens etwa 3 Zyklen ausgesetzt wird.

59. Verfahren nach Anspruch 1, wobei das Material wenigstens etwa 10 Zyklen ausgesetzt wird.

60. Verfahren nach Anspruch 1, wobei das Material wenigstens etwa 100 Zyklen ausgesetzt wird.

61. Herstellung eines Vakzins durch inaktivieren des Virus eimer Suspension eines Virus durch das Verfahren nach Anspruch 40.

## Revendications

1. Procédé de stérilisation d'une matière contenant un microbe, lequel procédé comporte :
- le fait de fournir une matière, sous une pression et à une température initiales ;
- le fait de refroidir la matière à une température Te, laquelle température Te vaut de -40 à 10 °C ;
- le fait d'exposer la matière à une pression hydrostatique élevée, suffisante pour inactiver au moins une partie des microbes contenus dans la matière ;
- le fait de faire baisser la pression jusqu'à une pression réduite, et de faire varier la pression, en cycles répétés, entre la pression élevée et la pression réduite ;
ce qui permet d'obtenir une matière stérilisée.

2. Procédé conforme à la revendication 1, dans lequel la matière comprend en outre une protéine, et la pression élevée n'est pas suffisante pour dénaturer cette protéine de manière irréversible.

3. Procédé conforme à la revendication 1, dans lequel la pression élevée est suffisante pour inactiver au moins à peu près 10 % des microbes contenus dans la matière.

4. Procédé conforme à la revendication 1, dans lequel la pression réduite vaut à peu près 1 atm.

5. Procédé conforme à la revendication 1, dans lequel la température initiale vaut à peu près 25 °C.

6. Procédé conforme à la revendication 1, dans lequel la température initiale est inférieure à environ 0 °C.

7. Procédé conforme à la revendication 6, dans lequel la température initiale vaut de -20 à -5 °C.

8. Procédé conforme à la revendication 1, dans lequel la pression élevée vaut à peu près de 34,5 à 655,0 MPa (5 000 à 95 000 psi).

9. Procédé conforme à la revendication 1, dans lequel la pression élevée vaut à peu près de 206,8 à 517,1 MPa (30 000 à 75 000 psi).

10. Procédé conforme à la revendication 1, dans lequel la pression élevée vaut à peu près de 655,0 à 1034,2 MPa (95 000 à 150 000 psi).

11. Procédé conforme à la revendication 1, qui comprend en outre le fait de refroidir la matière jusqu'à une température réduite avant les étapes d'augmentation de la pression et de variation de la pression en cycles.

12. Procédé conforme à la revendication 11, dans lequel ladite température réduite vaut à peu près de -40 à 0 °C.

13. Procédé conforme à la revendication 11, dans lequel ladite température réduite vaut à peu près de -20 à -5 °C.

14. Procédé conforme à la revendication 1, dans lequel la matière est une suspension de cellules pathogènes.

15. Procédé conforme à la revendication 14, qui comprend en outre le fait d'éliminer de la suspension pratiquement toutes les toxines qui peuvent se trouver présentes dans la suspension.

16. Procédé conforme à la revendication 1, dans lequel la température initiale vaut de -40 à 0 °C.

17. Procédé conforme à la revendication 1, qui comprend en outre le fait de laisser passer assez de temps pour que la température de la matière atteigne l'équilibre avant chaque augmentation de la pression.

18. Procédé conforme à la revendication 1, qui comprend en outre le fait de faire refroidir la matière.

19. Procédé conforme à la revendication 18, dans lequel on effectue l'étape de refroidissement avant l'étape d'augmentation de la pression.

20. Procédé conforme à la revendication 18, dans lequel on effectue l'étape de refroidissement après l'étape d'augmentation de la pression, mais avant l'étape de réduction de la pression.

21. Procédé conforme à la revendication 18, dans lequel on refroidit la matière jusqu'à une température valant à peu près de -40 à 0 °C.

22. Procédé conforme à la revendication 18, dans lequel on refroidit la matière jusqu'à une température valant à peu près de -20 à -5 °C.

23. Procédé conforme à l'une des revendications 18 à 22, qui comprend en outre le fait de réchauffer la matière jusqu'à une température accrue avant l'étape de réduction de la pression.

24. Procédé conforme à l'une des revendications 18 à 22, qui comprend en outre le fait de réchauffer la matière jusqu'à une température accrue après l'étape de réduction de la pression.

25. Procédé conforme à la revendication 1, qui comprend en outre, avant l'étape d'augmentation de la pression, le fait d'ajuster le pH de la matière à une valeur supérieure à environ 10.

26. Procédé conforme à la revendication 1, qui comprend en outre, avant l'étape d'augmentation de la pression, le fait d'ajuster le pH de la matière à une valeur inférieure à environ 4.

27. Procédé conforme à la revendication 1, dans lequel la matière à stériliser est choisie dans l'ensemble que constituent un échantillon biologique, du plasma sanguin, des produits thérapeutiques ou diagnostiques dérivés du plasma sanguin, des fluides biologiques, des fluides médicaux, des médicaments, des réactifs et des solutions de recherche, du sérum, un tissu vivant, un équipement médical ou militaire, un aliment, une préparation pharmaceutique, et un vaccin.

28. Procédé conforme à la revendication 1, dans lequel le microbe comprend un ou plusieurs éléments de l'ensemble constitué par les bactéries, virus, champignons, protistes, sporogènes, parasites protozoaires, parasites helminthiques, organismes vecteurs du paludisme, giardia, et cellules infectées par un virus.

29. Procédé conforme à la revendication 1, dans lequel on ajoute à la matière, avant de la stériliser, un catalyseur de transfert de phase.

30. Procédé conforme à la revendication 1, dans lequel on ajoute à la matière, avant de la stériliser, un agent de stabilisation de protéines.

31. Procédé conforme à la revendication 30, dans lequel l'agent de stabilisation de protéines est choisi dans l'ensemble formé par les sucres, le glycérol, un polymère hydrophile, une cyclodextrine, un caprylate, un acétyl-tryptophanate, un polyéthylène-glycol, un anti-oxydant et un ligand spécifique de protéine.

32. Procédé conforme à la revendication 1, dans lequel on ajoute à la matière, avant de la stériliser, un composé se liant à un acide nucléique.

33. Procédé conforme à la revendication 32, dans lequel le composé se liant à un acide nucléique est un agent de photosensibilisation.

34. Procédé conforme à la revendication 33, dans lequel l'agent de photosensibilisation est un psoralène.

35. Procédé conforme à la revendication 1, dans lequel la matière à stériliser est fournie dans son emballage final, lequel emballage est adapté pour pouvoir transmettre une pression sans se rompre.

36. Procédé conforme à la revendication 35, dans lequel l'emballage est hermétiquement enfermé dans du plastique souple.

37. Procédé conforme à la revendication 35, dans lequel l'emballage est une seringue et la pression est transmise par un piston.

38. Procédé conforme à la revendication 1, dans lequel l'étape où on fournit la matière comprend
- le fait d'introduire la matière dans un récipient adapté pour pouvoir transmettre à la matière une pression extérieure,
- et le fait de plonger le récipient dans un milieu de pressurisation, avant d'augmenter la pression.

39. Procédé conforme à la revendication 38, dans lequel le milieu de pressurisation est une solution d'un agent stérilisant choisi dans l'ensemble que constituent un agent oxydant, un alcool, de l'urée, un sel de guanidinium, un acide et une base.

40. Procédé conforme à la revendication 1, dans lequel le microbe est un virus.

41. Procédé conforme à la revendication 1 , dans lequel la matière contient une protéine, et la pression élevée est suffisante pour dénaturer de manière irréversible pratiquement toute cette protéine, si l'on maintient cette pression élevée pendant un laps de temps sensiblement plus long qu'un certain laps de temps tₑ, mais la pression élevée est insuffisante pour dénaturer de manière irréversible cette protéine, si l'on maintient cette pression élevée pendant un laps de temps inférieur ou égal à tₑ.

42. Procédé conforme à la revendication 41, dans lequel la pression élevée est suffisante pour dénaturer pratiquement toute la protéine, si l'on maintient cette pression élevée pendant un laps de temps plus long que 10 fois tₑ.

43. Procédé conforme à la revendication 41, dans lequel la pression élevée est suffisante pour dénaturer pratiquement toute la protéine, si l'on maintient cette pression élevée pendant un laps de temps plus long que 3 fois tₑ,

44. Procédé conforme à la revendication 41, dans lequel la protéine comprend un ou plusieurs facteurs de coagulation sanguine.

45. Procédé conforme à la revendication 41, dans lequel la protéine comprend une ou plusieurs immunoglobulines.

46. Procédé conforme à la revendication 41, dans lequel la protéine comprend une ou plusieurs protéines monomères.

47. Procédé conforme à la revendication 41, dans lequel la protéine comprend une ou plusieurs protéines multimères.

48. Procédé conforme à la revendication 41, dans lequel la matière est choisie dans l'ensemble que constituent du plasma sanguin, des produits thérapeutiques ou diagnostiques dérivés du plasma sanguin, des fluides biologiques, des fluides médicaux, des médicaments, des solutions de recherche, un tissu vivant, et des préparations pharmaceutiques.

49. Procédé conforme à la revendication 40, dans lequel le virus comprend un virus non-encapsulé.

50. Procédé conforme à la revendication 40, dans lequel le virus est choisi dans l'ensemble que constituent le parvovirus humain B19, le parvovirus porcin, le parvovirus bovin, le virus de l'immunodéficience humaine, le virus de l'herpès simplex, le virus de l'hépatite A et les virus transmissibles lors d'une transfusion.

51. Procédé conforme à la revendication 41, dans lequel la pression élevée vaut de 68,9 à 827,4 MPa (10 000 à 120 000 psi).

52. Procédé conforme à la revendication 41, dans lequel la pression élevée vaut de 275,8 à 689,5 MPa (40 000 à 100 000 psi).

53. Procédé conforme à la revendication 41, dans lequel la pression élevée vaut de 482,6 à 620,5 MPa (70 000 à 90 000 psi).

54. Procédé conforme à la revendication 1, dans lequel la pression réduite se situe entre la pression initiale et la pression élevée.

55. Procédé conforme à la revendication 41, dans lequel le laps de temps tₑ vaut de 0,5 à 300 secondes.

56. Procédé conforme à la revendication 55, dans lequel le laps de temps tₑ vaut de 10 à 30 secondes.

57. Procédé conforme à la revendication 1, dans lequel la matière subit de 2 à 100 cycles.

58. Procédé conforme à la revendication 1, dans lequel la matière subit au moins environ 3 cycles.

59. Procédé conforme à la revendication 1, dans lequel la matière subit au moins environ 10 cycles.

60. Procédé conforme à la revendication 1, dans lequel la matière subit au moins environ 100 cycles.

61. Production d'un vaccin par inactivation du virus d'une suspension de virus, réalisée selon un procédé conforme à la revendication 40.
